# EUROPEAN PATENT SPECIFICATION

(11) **EP 1 141 265 B1**
(45) Date of publication and mention of the grant of the patent: **03.09.2008**
(21) Application number: 00900283.3
(22) Date of filing: 13.01.2000
(51) Int. Cl.: C12N 15/00, A01K 67/027, A61K 48/00, C12N 5/10, C12N 5/06

(54) **DOUBLE NUCLEAR TRANSFER METHOD AND RESULTS THEREOF**
DOPPELKERNTRANSFERSVERFAHREN UND DESSEN ERGEBNISSE
PROCEDE DE DOUBLE TRANSFERT DE NOYAU ET RESULTATS DE CE TRANSFERT

(30) Priority: 13.01.1999 GB 9900734; 22.04.1999 US 130546 P
(43) Date of publication of application: 10.10.2001
(73) Proprietor: REVIVICOR, INC., Blacksburg, VA 24060 (US)
(72) Inventor: Campbell, Keith H. S., University of Nottingham, Loughborough, Leicestershire LE12 5RD (GB)
(74) Representative: Howard, Paul Nicholas
(86) International application number: PCT/GB2000/000086
(87) International publication number: WO 2000/042174

(56) References cited:
- WO-A-97/07668
- WO-A-97/07669
- WO-A-98/39416
- WO-A-98/57538

## Description

A method is described for the production of an animal (offspring) by the process of nuclear transfer. The animal may be produced using donor genetic material in cells taken directly from any stage of an animal, such as an embryo, foetus or adult or from cell cultures established from material from any stage of an animal, such as embryonic, foetal or adult material. The process may be used to introduce genetic modifications into the resultant offspring by genetic manipulation and selection of the cells to act as nuclear donors prior to embryo reconstruction. In addition, the process may be used to produce animals containing multiple genetic modifications by a number of routes including but not limited to; i) introduction of multiple genetic modifications concomitantly into the cultured cell population ii) repeated rounds of transfection and selection of the cultured cell population iii) repeated rounds of transfection, selection, nuclear transfer and re-isolation of a cell population from the embryo, foetus, juvenile or adult animal so formed iv) or any combination thereof. The process may be used to de-differentiate cells (including embryonic, foetal or adult somatic cells) for the production of undifferentiated cells, Embryonic Stem cells, other stem cell populations, germ cells or their derivatives or differentiated somatic cell populations preferably from embryonic foetal or adult stages of nuclear transfer reconstructed embryos.

The technique of nuclear transfer allows the production of offspring by the reconstruction of an animal embryo (which throughout this text includes all concepts of an animal embryo such as an oocyte, egg, zygote or an early embryo). Genetic material from a donor cell (karyoplast) is transferred to a suitable recipient cell from which the nuclear or genomic genetic material has been removed. In the first demonstrations of this technique successful development was only obtained when the donor genetic material was taken from undifferentiated cells or blastomeres from early embryos. Subsequently, development has been obtained using donor genetic material from differentiated cells maintained in culture and isolated from embryonic (Campbell et al. Nature (1996) 380:64-66), foetal and adult tissues (Wilmut et al Nature (1997) 385: 810-813) and in WO 97/07669 and WO 97/07668. Subsequently live offspring have been obtained in the mouse using quiescent cell populations derived directly *ex vivo* as nuclear donors (Wakayama et al., Nature (1998) 394:369-373). The successful use of differentiated cells has now been particularly demonstrated in sheep, cattle and mice.

The use of nuclear transfer (NT) technology has many benefits and uses in the production of mammalian embryos, foetuses and offspring. These include but are not limited to;
1. The ability to carry out precise genetic modification of cultured cells to be used as nuclear donors prior to embryo reconstruction. Such modifications include but are not limited to, random gene addition, addition of multiple copies of a transgene, addition of a transgene at a precise location (targeted addition or knockin), gene removal (knockout), gene inactivation by targeted insertion, gene replacement, modification of any gene or its control sequences and gene multiplication.
2. The ability to carry out multiple genetic modifications in a single animal either by multiple genetic modifications of a cell population in culture or by sequential genetic modification, nuclear transfer and re-isolation of a cell population from the embryo, foetus or animal so produced.
3. The ability to increase the lifespan of cultured cell populations to be used for genetic modification by nuclear transfer and re-isolation of a cell population from the embryo, foetus, juvenile or adult animal so produced.
4. The ability to produce multiple copies of an animal from a genetically modified selected and cloned cell population.
5. The ability to produce multiple copies of any embryo, foetus, juvenile or adult animal by nuclear transfer from cells taken directly *ex vivo* or cell populations derived from any tissues taken from any of these stages with or without culture *in vitro.*
6. The ability to produce true clones by utilising oocytes from the maternal line of the cell donor as cytoplast recipients for embryo reconstruction
7. The ability to store intact genomes for long periods (e.g. by freezing cell populations in liquid N2) and to subsequently use these stored cells for the production of offspring by nuclear transfer.
8. The ability to dedifferentiate somatic nuclei and to produce undifferentiated cells that may be used for production of chimeric embryos, foetuses and adult animals by embryo aggregation or injection or to produce embryonic stem or embryonic germ cell populations.
9. The ability to dedifferentiate any somatic cell type by nuclear transfer and to isolate from the embryo so produced embryonic stem cells, germ cells or any other desired specialised or unspecialised cell type e.g. neurones.

Genetic modification of animals and the production of stem cell and differentiated cell populations by nuclear transfer technology have numerous uses in the fields of human medicine, agriculture, genetic preservation and research. These include but are not limited to the production of human therapeutic proteins in bodily fluids, disease prevention, increasing required production traits, cell based therapies, cell based delivery systems for genetic therapy , tissue and organ transplantation. The uses of such technology have been previously discussed in many manuscripts and patents. Two of these are WO 98/30683 and WO 98/39416. The present invention is applicable to all stages of animals (cells, tissues, embryos and whole animals), in particular animals of an ungulate species including, a cow, bull, pig, goat, sheep, camel or water buffalo, as well as a mouse, rat or other rodent, and also lagomorph animals, such as a rabbit. The invention does not relate to human reproductive cloning. Thus the "embryo", "oocyte", "zygote", "donor nucleus", "somatic cell", "diploid cell", "diploid nucleus" and "recipient cell" used with the invention do not include human cells, nuclei or embryos."

In general, oocytes arrested at metaphase of the second meiotic division have been used as cytoplast recipients. The donor genetic material has been introduced into the recipient cell cytoplasm by the processes of 1) cell fusion 2) injection of intact cells lysed cells or nuclei. Transfer of genetic material may occur either at the time of activation, preceding activation (see WO 97/07669 and WO 97/07668 or following activation (Campbell et al., Biol. Reprod. 49.9330942 (1993); Campbell et al Biol. Reprod. 50 1385-1393 (1994)). In each of these instances the ploidy of the reconstructed embryo must be maintained by the use of donor genetic material at an appropriate stage of the cell cycle (for review see Campbell et al., Reviews of Reproduction 1:40-46 (1996)). This process may be coupled with genetic manipulation techniques for the production of transgenic offspring (Schnieke et al., Science 278:2130-2133 (1997)). The use of nuclear transfer coupled to genetic modification of cells in culture and their selection prior to animal production has a number of advantages including:
1. production of non-mosaic animals ensuring germ line transmission of the genetic modification/s
2. An increased efficiency in the production of such genetically modified animals.
3. The production of multiple copies of the offspring thereby reducing the generation interval to produce flocks or herds for production purposes or increasing the numbers of animals for dissemination of genetic modification into the population as a whole.
4. The production of animals containing multiple genetic modifications. ,
5. The production of transgenic animals with superior expression characteristics by utilisation of the pre-selection of the integration site of the transgene.

In pigs, the use of nuclear transfer for the production of live offspring has been associated with difficulties. Although offspring have been produced by swapping the pronuclei of fertilised zygotes, only 1 piglet has been produced from a later developmental stage (4 cell) (Prather et al., Biol Reprod 1989 Sep 41:3 414-8).

The process of embryo reconstruction and production of viable offspring by nuclear transfer is a multistep procedure. Each of these steps will now be described in more detail prior to a description of the present invention as a whole. The steps are those included in the present invention. The description below is intended to give explanation to the steps and is not an exhaustive nor limiting description.

### The Recipient Cell or Cytoplast.

Oocytes, fertilised zygotes and two cell embryos have been used as cytoplast recipients for nuclear transfer. In general oocytes arrested at metaphase of the second meiotic division (also termed unfertilised eggs) have become the cytoplast of choice. At this point in oocyte development the genetic material is arranged upon the meiotic spindle and is easily removed using mechanical means. Several reports have demonstrated that during maturation i.e. between the germinal vesicle stage (prophase of the first meiotic division) and arrest at metaphase of the second meiotic division genomic DNA can be removed and the resulting cytoplast used for nuclear transfer (Kato Y., Tsunoda Y., Mol Reprod Dev (1993) Oct 36:2 276-8). The use of fertilised zygotes as cytoplast recipients has been reported in mouse (Kwon O.Y., Kono T., Proc Natl Acad Sci U.S.A (1996) Nov 12, 93:23 13010-3), cattle (Prather R.S., First N.L., J Reprod Fertil Suppl (1990) 41:), and pigs (Prather et al., Biol Reprod (1989) Sep 41:3, 414-8). In cattle and pigs, development of embryos reconstructed using zygotes as cytoplast recipients is low and on the whole restricted to the exchange of pronuclei, suggesting that factors essential for successful development are removed with the pronuclei. The subject matter of the present invention is to produce a pronucleus-like structure (including a pronucleus) by transfer of a suitable nucleus, preferably to an enucleated Metaphase II oocyte. The pronucleus-like structure thus formed will contain factors, which are generally removed during zygote enucleation. This pronucleus-like structure is then used as a donor of genetic material for a second nuclear transfer embryo reconstruction. Nuclei or cells of any cell cycle stage may be used as donors of genetic material preferably however; the cell cycle stage of the recipient cytoplast must be controlled accordingly.

### Preparation of a Cytoplast Recipient by Removal of the Genomic Genetic Material.

This process has in general been termed enucleation. In the majority of recipients utilised, the genomic DNA is not enclosed within a nuclear membrane at the time of removal. The removal of the genetic material according to the present invention and described by the term "enucleation" does not require that the genetic material is present in a nuclear membrane (it may or may not be, or may partially be). Enucleation may be achieved physically by actual removal of the nucleus, pronuclei or metaphase plate (depending on the recipient cell), or functionally, such as by the application of ultra-violet radiation or another enucleating influence. Removal of the genetic material is possible by physical and/or chemical means. In the early reports of nuclear transfer, MII oocytes were simply cut in half on the basis that one half would contain the genetic material and the other would not. Modifications to this approach have been made in order to reduce the volume of cytoplasm, which was removed. This may be achieved by aspiration of a small amount of cytoplasm from directly beneath the first polar body using glass micropipettes or by using a knife to cut away that part of the oocyte beneath the polar body. To facilitate plasticity of the oocyte it may be pre-treated with the microtubule inhibitor Cytochalasin B or other such agent that disrupts the cytoskeleton. In contrast to physical enucleation, chemical treatment has been demonstrated to cause complete removal of the genetic material in the mouse.

Treatment of maturing oocytes with the Topoisomerase inhibitor ectoposide results in the expulsion of all genomic material with the first polar body (Elsheikh A.S. et al Jpn J Vet Res (1998) Feb 45:4, 217-20), however no development to term has been described using cytoplast recipients produced by this method and there are no reports of this procedure in other species. Centrifugation of MII oocytes combined with Cytochalasin B treatment has been reported to cause enucleation in hamster and cattle oocytes (Tatham et al., Hum Reprod (1996) Jul 11:7 1499-503). The development of embryos reconstructed from such cytoplasts has been reported in cattle however the frequency of development is low.

When using zygotes, the genetic material may be removed by mechanical aspiration of both pronuclei. Dependent upon species, in order to facilitate visualisation of the pronuclei the zygotes may be centrifuged prior to enucleation.

### Introduction of genetic material (embryo reconstruction).

Having prepared a suitable recipient cell or cytoplast the donor genetic material must be introduced. Various techniques have been reported including;
1. Cell fusion induced by chemical, viral or electrical means.
2. Injection of an intact cell by any method
3. Injection of a lysed or damaged cell.
4. Injection of a nucleus.
Any of these methods may be used in any species with some modifications of individual protocols.

### Activation of the reconstructed embryo.

In addition to the transfer of donor genetic material from the karyoplast to the cytoplast, the cytoplast must be stimulated to initiate development. When using a fertilised zygote as a cytoplast recipient development has already been initiated by sperm entry at fertilisation. When using MII oocytes as cytoplast recipients the oocyte must be activated by other stimuli. Various treatments have been reported to induce oocyte activation and promote early embryonic development including, but not limited to; application of a DC electric stimulus, treatment with ethanol, ionomycin, Inositol tris-phosphate (IP₃), calcium ionophore A23187, treatment with extracts of sperm or any other treatment which induces calcium entry into the oocyte or release of internal calcium stores and results in initiation of development. In addition any of these treatments, alone or in combination, their application at the same or different times or in combination with inhibitors of protein synthesis (i.e. cycloheximide or puromycin) or inhibitors of serine threonine protein kinases (i.e. 6-DMAP) may be applied.

### Culture of reconstructed embryos.

Nuclear transfer reconstructed embryos may be cultured *in vitro* to a stage suitable for transfer to a final recipient using any suitable culture medium or culture process. Alternatively, embryos may be cultured *in vivo* in the ligated oviduct of a suitable host animal (in general sheep) until a stage suitable for transfer to a final surrogate recipient is reached. Embryos from cattle, sheep and other species may be cultured in a trans species recipient, for simplicity a sheep provides a suitable recipient for bovine, ovine and porcine species. In order to prevent mechanical damage or attack by macrophages to the reconstructed embryos whilst in the oviduct of the temporary recipient it is usual to embed the embryos in a protective layer of agar or similar material.

The *in vitro* culture systems presently available for porcine embryos support development to the blastocyst stage, however, the frequency of live births from such embryos is low (Machaty et al., Biology of Reproduction, (1998) 59, 451-455). In the present invention the first nuclear transfer reconstructed embryo may be cultured by any of these methods until the formation of a pronuclear-like body occurs and the embryo is used for the second nuclear transfer reconstruction. In practice it is presently preferred that this first culture period is carried out using a suitable medium *in vitro.*

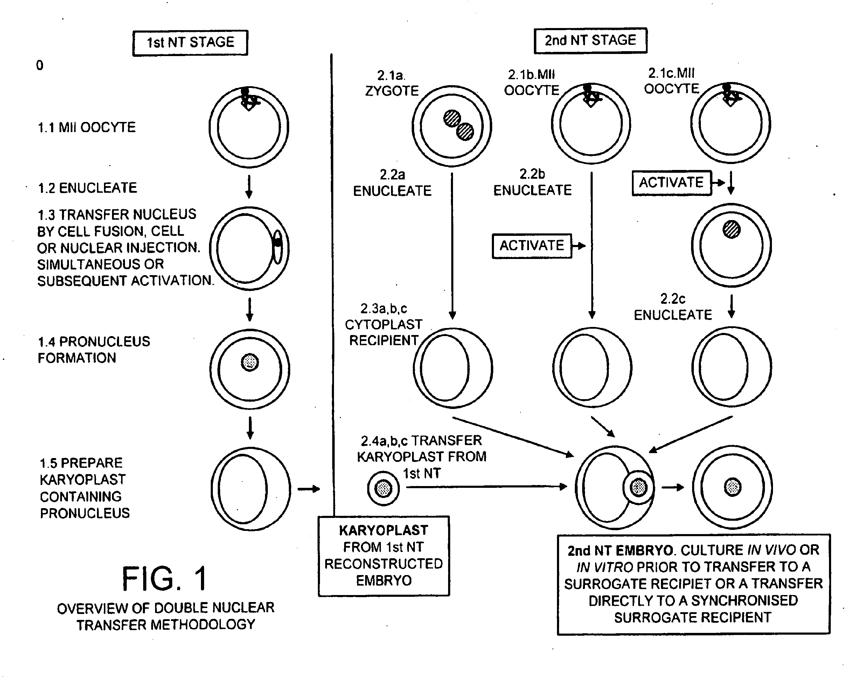

Each of the steps involved in the reconstruction of mammalian embryos by nuclear transfer is inefficient. In pigs this is particularly evident by the lack of reports on the production of live offspring from nuclear transfer reconstructed embryos. The basis of the present invention is to minimise the inefficient steps involved in the reconstruction process using a multistep embryo reconstruction procedure and culturing the embryos in the final surrogate recipient. Accordingly, one aspect of the invention provides a method of reconstituting an animal embryo. The process comprising transferring a nucleus into a first oocyte followed by removing and transferring said nucleus from said oocyte to a further recipient cell. Preferably, the nucleus transferred from the first oocyte is in the form of a pronucleus-like structure. Preferably, the second recipient cell is an oocyte or an enucleated fertilised zygote. This involves a double nuclear transfer procedure; The first step of this procedure is to produce a pronucleus-like structure by transfer of a donor nucleus to an oocyte. Preferably the oocyte is a mature metaphase II oocyte (unfertilised egg), which has been previously or is simultaneously or subsequently activated, or an activated MII oocyte (the activation is usually by physical or chemical input). An example of the first step is shown in Figure 1 (for simplicities sake, the use of an activated recipient in Figure 1 is not shown, but this is documented in Table 1). In the second step of the procedure the pronucleus-like strucure generated by the first step is removed from the first reconstructed embryo and transferred to an oocyte or to an activated parthenote or enucleated fertilised zygote (Figure 1). Where the transfer is to an oocyte, preferably the pronucleus-like structure is removed from the first reconstructed embryo and transferred to an oocyte which is an enucleated MII oocyte prior to, concomitant with or following activation. Following the second nuclear transfer the reconstructed embryo may be transferred directly to a final surrogate recipient for development to term.

The donor genetic material for embryo reconstruction can be provided by any differentiated, partially differentiated or undifferentiated cell taken directly from any stage in an animal development, such as an embryo, foetus, juvenile or adult animal or any cell line derived from such. In previous reports the cell cycle stage of the donor nucleus has been shown to be critical for development. In the present invention a cell at any stage of the cell cycle can be used as donor of genetic material for the first reconstruction process. However, the combination of cell cycle stages of the donor nucleus and the recipient cytoplasm will vary dependent upon the cell cycle stage of the cell to be used as donor of genetic material at the time of transfer. The donor genetic material and the recipient cells, are not required to be from the same animal species, although this may be preferred.

### PREPARATION OF DONOR CELLS.

Methods and materials for the culture, synchronisation and selection of donor cells to be used as nuclear donors for the reconstruction of embryos by nuclear transfer are well known to anyone skilled in the art. Any cell population obtained directly from any stage in the life of an animal (for example, embryo, foetus or adult animal) may be used as a donor genetic material, alternatively a cell from any population may be used (for example an undifferentiated, partially differentiated or differentiated cell population) derived from any stage (for example embryo, foetus, juvenile or adult animal) and maintained in culture or any cell (for example undifferentiated, partially differentiated or differentiated cell) resulting during culture of primary isolates or as a result of treatment of the cultured cell population with hormones, growth factors or chemicals which alter the differentiated state. The cell cycle stage of cells maintained in culture may be manipulated to ensure coordination with the recipient cytoplast using any means available to any one skilled in the art. The following examples are non-limiting and are merely representative of various aspects of the art.

### G0-phase

Any method which results in the production of quiescent (non-growing) diploid cells which have arrested the cell cycle following mitosis and cell division but prior to the onset of DNA Replication or S-phase may be used. Suitable methods include but are not restricted to;
I. Quiescence induced by contact inhibition of cultured cell populations
II. Quiescence induced by removal or reduction in concentration of serum or other essential nutrient in the culture medium i.e. leucine or other amino acid, specific growth factor/s.
III. Quiescence induced by senescence
IV. Quiescence induced by addition of a specific growth factor or other substance.
V Quiescence induced by any physical means such as heat shock, hyperbaric pressure or treatment with chemical compounds which induce a heat shock or stress response e.g puromycin, azacytidine.

### G1-phase

Donor nuclei may be arrested in the G1 phase of the cell cycle by treatment with any chemical, hormone, growth factor or other substance which causes arrest or by any physical stress which induces cell cycle arrest i.e. induction of a heat shock response as a result or elevated temperature or treatment with compounds which induce such a response i.e. puromycin, azacytidine. Alternatively G1 phase cells may be obtained by synchronisation of the cell population by any means and selection of G1 cells at an appropriate time. For example cells may be arrested in the M-phase of the cell cycle by treatment with microtubule disrupting drugs such as Colcemid or Nocodazole, mitotic cells may then be selected from the population by shake-off and placed into a separate culture vessel. G1 cells may then be selected at an appropriate time following shake off. As a variation of this technique, mitotic cells may also be selected from an unsynchronised population. G1 cells may be enriched by causing cells to exit the growth cycle and arrest in a quiescent or G0 phase (as above) and then restimulating growth by re-addition of the restricting factor. G1 cells can then be selected at a time following restimulation to be found by experimentation for each individual cell type/population. This technique may be combined with any technique which then arrests cells in either the G1 phase of the cell cycle, or at the G1/S-phase border i.e. treatment with hydroxyurea or aphidicolin.

### S-phase.

S-phase cells can be sele10cted or enriched for by treatment with any chemical agent which interferes with any point of the replication procedure e.g. aphidicolin which inhibits chain elongation in a concentration dependent manner, Hydroxyurea which inhibits the enzyme ribonucleotide reductase. Cells synchronised in any other stage of the cell cycle may be released and the timing of S-phase after release determined experimentally. In addition these two processes may be combined or any other selection or synchronisation procedure or combination thereof utilised.

### G2-phase

G2 phase cells may be selected from a growing population that has been synchronised by any physical, chemical or selective method. Alternatively G2 cells may be enriched for by treatment of a synchronised or unsynchronised cell population with any agents that specifically arrest the cell cycle following the completion of S-phase and prior to the onset of mitosis.

### M-phase

Mitotic cells may be selected from an unsynchronised population using any means possible including but not being limited to, mitotic shake off, elutriation, FAC's (fluorescence activated cell sorting). Alternatively cells may be arrested in mitosis by treatment with microtubule disrupting agents such as colcemid or nocodazole, by treatment with DMAP or any other serine threonine protein kinase inhibitors or by any other chemical or physical means which disrupts normal growth progression and causes arrest in mitosis.

### Unsynchronised cell populations.

Unsynchronised cell populations may be obtained from any embryonic foetal or adult tissue and cultured *in vitro* or any cell removed directly from an embryo, foetus, juvenile or adult animal. An unsynchronised cell is defined as a cell, the cell cycle stage of which is unknown at the time of transfer to a recipient cytoplast.

### NUCLEAR TRANSFER.

### I) Oocytes.

Oocytes to act as suitable cytoplast recipients can be obtained using a variety of techniques and protocols, these include but are not limited to
i) *In vitro* maturation following follicle aspiration from ovaries obtained at slaughter or removed surgically.
ii) Transvaginal follicle puncture followed by maturation *in vitro*
iii) *In vivo* matured but collected by transvaginal follicle puncture prior to ovulation, maturation is then *completed in vitro.*
iv) *In vivo* matured and surgically recovered.
v) *In vivo* matured and recovered at slaughter.

It is preferable, but not essential, that all *in vivo* matured oocytes are harvested by flushing from the oviduct in calcium magnesium free phosphate buffered saline (PBS) (or other suitable medium devoid of calcium and magnesium ions) containing 1.0% foetal calf serum (FCS). Similarly *in vitro* matured oocytes are harvested and transferred to suitable medium devoid of calcium and magnesium ions (for example M2 medium prepared without addition of calcium and magnesium ions). The chosen medium may be supplemented with FCS, BSA or other protein source. Oocytes are denuded of cumulus cells and enucleated as previously described (Campbell *et al.,* 1993.1994) with the exception that calcium free medium, although not essential is preferred for all procedures. Fusion procedures are modifications of those previously reported (Campbell *et al.,* 1993,1994) and are as described in the relevant section below, alternatively the nucleus may be introduced by manual injection of the donor cell into the enucleated oocyte (Ritchie and Campbell, J. Reproduction and Fertility Abstract Series No. 15, p60) or by using a commercially available piezzo injection apparatus as demonstrated in the mouse (Wakayama *et al.,* 1998). The timing of these events is dependent upon the species. The protocols outlined in this application define the use of *in vivo* and *in vitro* matured ovine, bovine and porcine oocytes coupled to the use *of in vitro* or *in vivo* produced zygotes. The procedure is not limited to these defined protocols.

### II) Zygotes.

To act as recipients for the second nuclear transfer fertilised zygotes or oocytes (prior to, concomitant with or following activation) are required. At the present time fertilised zygotes are preferred, although not essential. Matured oocytes may be obtained by any of the procedures outlined above. The genetic material may be removed from the oocyte as previously described. Oocytes may be activated by treating the matured oocytes with any physical or chemical stimulus or combination thereof, which causes activation and stimulates development. The timing of application of the activation stimulus will vary somewhat upon both the species and the method of maturation. Fertilised zygotes may be obtained by *in vitro* fertilisation of matured oocytes obtained from any of the above sources or alternatively recovered *ex vivo* following mating or A1 using stimulated or non-stimulated donor animals.

### FIRST STAGE NUCLEAR TRANSFER EMBRYO RECONSTRUCTION.

Donor cells at different stages of the cell cycle may be used for embryo reconstruction. The cytoplast recipient used and any additional treatments required will vary somewhat, dependent upon the cell cycle stage of the donor and recipient cells. Table 1 gives a series of possible combinations.

In fact, any method may be used which results in the production of one or more pronucleus-like structure, preferably of normal diploidy or tetraploidy.

### SECOND STAGE NUCLEAR TRANSFER:

In the second stage of the invention a pronucleus-like structure (preferably identified as a swollen form of the nucleus) from the first nuclear transfer reconstructed embryo is transferred to an enucleated MII (prior to, concomitant with or following activation) or to an activated parthenote or fertilised zygote which have been enucleated by removal of the pronuclei. Although each of these cytoplast recipients are suitable for the second nuclear transfer in practice an enucleated fertilised zygote is the preferred choice. The zygote to act as cytoplast recipient for the second nuclear transfer may be produced *in vivo* or *in vitro,* although in practice the use of *in vivo* produced zygotes is the method of choice at present. Alternatively any cell resulting from development of the first nuclear transfer embryo or cultured cell population derived from this embryo may be used as a donor of genetic material for production of the second nuclear transfer embryo.

### Source of activated oocyte or zygote:

Oocytes obtained from any *in vitro* or *in vivo* source and subsequently matured *in vitro,* or oocytes matured *in vivo* may be used as recipients for the second nuclear transfer following enucleation and subsequent activation or activation and subsequent enucleation. In practice it is preferred that the recipient cytoplast for the second nuclear transfer is prepared from a fertilised zygote. The zygote required for production of a cytoplast recipient for the second nuclear transfer may be obtained from any suitable source which may include but is not limited to; *In vivo* produced fertilised zygotes that are recovered from a suitable donor animal. Zygotes may be produced by natural mating, by natural mating following superovulation or by AI using fresh or frozen semen. Alternatively, fertilised zygotes may be produced *in vitro. In vitro* fertilisation may be carried out on oocytes matured *in vivo* from suitable donors with or without superovulation or by using oocytes matured *in vitro* following recovery from slaughterhouse material or aspiration of follicles *in vivo.* In practice it is preferred that the zygote for the second nuclear transfer is obtained following maturation and fertilisation *in vivo.*

### Enucleation of zygotes.

Following successful fertilisation the male and female chromatin in the newly formed zygote decondenses and forms two pronuclei. The genetic material may be removed at any point following fertilisation. However, in practice the present method of choice is to remove the genetic material after formation of the pronuclei. Due to the nature of the cytoplasm it is often difficult to visualise and remove the pronuclei from a fertilised zygote. Visualisation of the pronuclei may be aided by centrifugation of the zygotes prior to their manipulation, although this is not essential.

### Preparation of a karyoplast donor from first nuclear transfer embryo

The pronucleus/i formed in the first nuclear transfer reconstructed embryos must be removed and transferred to the second cytoplast recipient. To visualise the pronucleus/i the embryos are preferably centrifuged. Aspiration of a pronucleus surrounded by cytoplasm and enveloped in a cell membrane is then carried out, preferably by microsurgery. To facilitate this process, although not a necessity, the embryo may be rendered more *pliable* by treatment with one or more chemical compounds, which disrupt the cytoskeleton e.g. Cytochalasin B, or Nocodazole. The centrifuged embryo is preincubated in the agent/s of choice and then transferred to a manipulation chamber. Using a fine glass pipette a karyoplast is aspirated which contains a pronucleus.

### Reconstruction of second nuclear transfer embryo

The karyoplast formed from the first nuclear transfer embryo is used to transfer the genetic material now contained within the pronucleus like structure to the enucleated second recipient cytoplast. Any means of transfer including fusion of the karyoplast to the cytoplast by viral, chemical or electrical means may be used or alternatively the pronucleus may be injected into the recipient cell. Due to the size of the pronucleus it is preferred but not essential that the genetic material is transferred by the process of cell fusion. For cell fusion the karyoplast is transferred below the zona pellucida of the second cytoplast recipient and placed into contact with the enucleated zygote. Any physical, chemical or viral means of inducing cell fusion may then be applied. However, at the present time it is preferable, but not essential, to use electrical fusion. For electrical fusion the couplet is placed into a suitable medium in a suitable fusion chamber. A DC electrical pulse is then passed through the couplet at right angles to the plane of contact between the cytoplast and karyoplast. An AC electrical pulse may first be used to align and/or increase contact between the cytoplast and karyoplast. The duration strength and timing of the electrical pulse/s all affect the frequency of fusion and can be optimised by experimentation.

### Culture of second nuclear transfer embryos.

Following reconstruction of the second nuclear transfer embryo, further development may be carried out by culturing in suitable medium *in vitro* until a developmental stage suitable for transfer to a final surrogate recipient is reached. Alternatively, reconstructed embryos may be cultured in the ligated oviduct of a suitable, synchronised, intermediate recipient for sufficient time to reach a developmental stage suitable for transfer to a final surrogate recipient. Or as is the method of choice at present in porcine species, the second nuclear transfer reconstructed embryo can be transferred directly to the final surrogate recipient and pregnancy maintained by hormonal treatment (see below).

### Transfer to final surrogate recipient

Reconstructed embryos may be cultured by any means *in vitro* or *in vivo* to any stage suitable for transfer to a synchronised final recipient for development to term. Alternatively reconstructed embryos may be transferred as soon as possible to a synchronised recipient for development to term. The latter method is presently the method of choice in porcine species. In this situation induction and maintenance of pregnancy in the final recipient can be achieved by either of the following procedures;
At the time of transfer *in vivo* produced embryos at the same developmental stage as the nuclear transfer reconstructed embryos may be transferred with the nuclear transfer. Alternatively, the recipient may be treated with a combination of hormones in order to maintain pregnancy (Christenson et al., J. Animal Science 1971; 32:282-286).

The present invention encompasses genetic modification as known in the an to provide transgenic animals of all stages. The term "transgenic" refers broadly to any animal stage whose germ line has been the subject of technical intervention by recombinant DNA technology. This includes introduction of genetic material from another species as well as an animal in whose germ line an endogenous gene has been deleted, duplicated, activated or modified.

The genetic material (donor nucleus) can be modified at any stage in the double nuclear transfer methodology. Most preferably, the recombinant intervention is carried out before the first nuclear transfer step. The recombinant intervention may be carried out between the first and second nuclear transfer steps.

For recombinant intervention before the first nuclear transfer step, the genetic material is preferably modified as part of a cell line modification or taken from a previously modified animal. A level of selection can be introduced to identify donor nuclei for the nuclear transfer step. Alternatively, or in addition, recombinant intervention can be carried out after the first nuclear transfer step and before the second. In this scenario, the cell obtained may be genetically modified by the same or a different method.

Suitable recombinant technology for genetically modifying nuclear material is well known in the art. It includes methods as discussed and disclosed in WO97/07669, WO97/07668, US5,612,205, US5,721,367, WO99/01164, WO99/21415, US 5,945,577 and WO98/371

### The present invention includes the use of unenucleated oocytes as cytoplast recipients for embryo reconstruction by nuclear transfer.

The development of embryos reconstructed by nuclear transfer is dependent upon many factors that include the cell cycle stage of both the recipient cytoplasm and the donor nucleus. The ability to synchronise the blastomeres of early murine embryos has allowed detailed studies of cytoplast/karyoplast cell cycle combinations. Reports in the mouse have demonstrated the use of mitotically arrested cells as donors of genetic material (Kwon and Kono, . Proc. Natl. Acad. Sci. U. S. A.93, 13010-13013). Briefly; blastomeres from nocodazole treated embryos arrested in mitosis are transferred to enucleated MII oocytes. Extrusion of a polar body is inhibited by treatment with cytochalasin B, this results in the formation of two diploid (pro) nuclei. Each of these nuclei is then transferred individually to an enucleated zygote. Using this method, these authors produced 6 identical pups from a single 4-cell embryo. In a further study Otaegui and co-workers (unpublished) the results suggest that during the late G2 and early G1 phase of the donor cell cycle the chromatin is more able to re-direct development. Together these two studies suggest that during late G2, M and early G1 a permissive state exists which allows nuclear reprogramming. One possible explanation for these observations is that during these cell cycle phases, certain factors are displaced from the chromatin as a result of condensation, thus allowing access of oocyte derived factors. This hypothesis is supported by two lines of evidence; firstly, during mitosis, nascent transcripts and transcription factors become displaced from the chromatin (Schermoen, A.W.and O'Farrell, P. H. (1991) Cell.67, 303-310. Martinez-Balbas et al. , Cell.83, 29-38) and secondly the production of live offspring using mitotically arrested blastomeres as donors of genetic material (Kwon and Kono, . Proc. Natl. Acad. Sci. U. S. A.93, 13010-13013).

When using somatic cells as nuclear donors it has been shown that cells arrested in the G0 phase (quiescent) of the cell cycle are able to direct development to term of nuclear transfer reconstructed embryos. The chromatin of quiescent cells has been reported to undergo condensation, these cells also show a reduction in transcription and translation, mRNA is actively degraded and changes occur in the polyribosomes Whitfield, J. F., et al (1985) In'Control of Animal cell proliferation'. (Eds Boynton A. L. and Leffert, H. L.)pp331-365. (Academic Press Inc:London.) Quiescent cells reduce their metabolism to that which is required to maintain viability. Conceptually one may expect that the chromatin of cells arrested in G0 and poised to undergo differentiation may be more amenable to respond to a variety of signals. Alternatively, it may merely be a 'more null' state that allows the chromatin to respond to oocyte maternal cytoplasmic factors controlling gene expression after nuclear transfer. This hypothesis is supported by previous observations in the mouse using embryonic blastomeres as nuclear donors (see above). In these reports, donors in late G2-phase M-phase and nuclei in early G1-phase (in which chromatin decondensation is incomplete) have a greater developmental potential than late G1, S or early G2 donors. In these more successful donor nuclei, one can speculate that the release of transcription factors due to chromatin condensation in the donor cell then allows greater access of oocyte cytoplasmic factors as the chromatin decondenses after transfer. Further support for this hypothesis comes from studies in cultured cells which demonstrated that during mitosis, transcription factor binding affinity remains the same but these factors are excluded from the chromatin due to its condensation (Martinez-Balbas et al ., Cell.83, 29-38). More recently, other researchers have shown that transcriptionally inactive genes localise to centromeric heterochromatin in the nuclei of cycling but not quiescent primary B-lymphocytes. Stimulation of quiescent cells to re-enter the growth cycle causes repositioning of inactive loci to the centromeric regions ( Brown et al, Mol Cell Biol 3, 207-217). Thus the chromatin of quiescent cells may be more amenable to structural changes after nuclear transfer which are associated with 'reprogramming' of gene expression.

The ability to utilise different cell cycle combinations of donor and recipient cells is dependent upon species. In the mouse, an intact organised spindle is obtained when G2 or M phase nuclei are transferred into enucleated MII oocytes. On activation a "pseudo-mitotic/meiotic" event occurs; a polar body is extruded and a diploid nucleus formed. Alternatively suppression of polar body expulsion by treatment with Cytochalasin B results in a tetraploid embryo with 2 diploid pronuclei. Each of these pronuclei can be used (successfully) as a nuclear donor to an enucleated zygote. In cattle sheep and pigs, following transfer of a nucleus from a diploid (G1) blastomere or a somatic cell into an enucleated MII oocyte, no organised spindle is observed and there are no reports of polar body extrusion suggesting that no organised spindle is in fact formed. Similarly in cattle, when a G2 nucleus is transferred into an enucleated MII oocyte no organised spindle is observed, however if the oocyte is not enucleated 2 organised spindles are observed, one the original oocyte MII and the second organising the chromosomes derived from the donor nucleus.

These differences in spindle organisation can be used to advantage. Nuclei in the G2 or M-phases of the cell cycle can be transferred to a maturing oocyte either I) at a point following metaphase of the first meiotic division i.e. between MI and MII or ii) at MII. At MII there would be 2 spindles present 1 from the maternal DNA and the other from the transferred DNA. Activation of the recipient oocyte can be prevented by methods known in the art i.e. removal of calcium from the culture, manipulation or fusion medium. The transferred chromatin which is condensed into chromosomes may then be allowed to 'be conditioned' in the recipient cytoplasm for a period dependent upon the species and the age of the recipient oocyte. The maternal chromatin may be removed following formation of an intact spindle containing the donor chromatin, or alternatively following activation of the reconstructed oocyte. Activation may be achieved by methods common to the art with the exception that spindle integrity must be maintained. On activation the transferred DNA undergoes a pseudo- mitotic/meiotic division and extrudes a polar body thus giving a diploid embryo. Alternatively the extrusion of the polar body could be prevented with compounds common to the art e.g. Cytochalasin B and the resulting zygote would have 2 diploid pronuclei each of which could be used for transfer to an enucleated zygote or an activated enucleated oocyte.
The original oocyte or maternal DNA could be selectively removed following identification by a number of means
1. By observation of the 1^{st} polar body and aspiration of cytoplasm and the meiotic spindle from directly beneath the polar body prior to activation - this depends on the age of the recipient oocyte as the polar body breaks down on increasing age.
2. Either the maternal or donor chromatin may be selectively stained prior to reconstruction with a DNA specific dye that will then allow selective removal of the stained or unstained chromosomes prior to activation.
3. As in 3 with subsequent removal of the chromosomes or the subsequent pronucleus following activation.
4. By use of transgenic donor cells which contain a specific marker gene driven by a G0 specific promotor sequence.

### Examples of the use of unenucleated oocytes:

The protocols described for the production of oocytes as recipients for 1^{st} nuclear transfer procedures as described above are all suitable for the use of unenucleated oocytes. Transfer of the donor nucleus can be achieved as previously described using electrical, chemical or virally induced fusion or injection by any means including the use of a piezzo. Enucleation following transfer of the donor nucleus can be achieved by any methods known to the art. The timing of the various procedures will vary between species and be dependent upon the source of oocytes and will be determined experimentally.

The donor cells should be placed in a suitable DNA specific fluorochrome to stain the DNA - i.e. 1 microgram of Hoescht 3332 for 10 minutes in culture medium followed by incubation in medium lacking, the stain. Transfer of the nucleus can be carried out at any time after the appearance of telophase of the 1^{st} meiotic division which may be observed microscopically by the appearance of a small protuberance on the cell wall of the oocyte which is well known to those skilled in the art. Manipulation and reconstruction of the recipient oocytes is preferred in a medium that lacks calcium in order to prevent activation. The reconstructed oocytes can then be cultured until the maternal DNA is removed. Sufficient time is allowed for formation of the mitotic spindle (this will vary between species and culture conditions and also on the timing of reconstruction). In the case of Telophase 1 recipients sufficient time is allowed for the oocyte maternal chromatin to reach MII. Enucleation of the maternal chromatin may be achieved at MII by brief exposure to UV light for visualisation and identification of the stained chromatin. Alternatively, the oocytes may be activated and then exposed to UV for identification of the donor chromatin at any time prior to and following pronuclear formation. Following removal of the maternal chromatin and activation the presumptive zygotes are cultured by any means known to the art to a stage suitable for transfer to a synchronised final recipient for development to term.

The unenucleated oocyte is preferably used in the first nuclear transfer step. It may be used in the second.

### EXAMPLES

The examples below are non-limiting and are merely representative of various aspects of the present invention.

### I) PROTOCOL FOR PORCINE NUCLEAR TRANSFER.

### Synchronisation procedure for the collection of in vivo matured Porcine Oocytes.

A suitable procedure for the collection *of in* vivo matured porcine oocytes is described below. In practice any procedure which results in the production of suitable oocytes may be used.

Feed Altrenogest (Regu-Mate, 18 mg/day, 9 AM) for 5 - 9 days, starting on day 12-16 of the cycle (day 0 is heat). If no heats are available, feed Altrenogest for 14 days. Inject gilts with Estrumate (IM, 1.0 ml, 9 AM) during the last Alternogest feeding. Inject eCG (Equine Chorionic Gonadotropin, 1500 IU IM) 14 hrs (11 PM) after the last Altrenogest feeding. Inject hCG (human Chorionic Gonadotropin, 1000 IU, IM) 80 hrs after the eCG injection (7 AM). Collect oocytes 50 hrs after the hCG injection (9 AM).

### FIRST STAGE NUCLEAR TRANSFER EMBRYO RECONSTRUCTION.

A range of protocols are available for the first stage nuclear transfer. Their use is dictated by the cell cycle stage of the donor cell nucleus at the time of transfer.

### 1. "MAGIC" (Metaphase Arrested G1/G0 Accepting Cytoplast).

Enucleated oocytes were maintained in calcium free medium 10% FCS at 39°C. In the first stage nuclear transfer it is presently preferred, but not essential, that the donor genetic material is introduced into the enucleated oocyte by piezzo aided injection. To aid injection the cytoplast recipients may be centrifuged at 13,000xG for 5 minutes. The donor cell is placed into medium containing 12% Polyvinyl Pyrollidone (optional), the cell is drawn into the injection pipette and then injected into the oocyte, the cell membrane may be intentionally damaged by selection of an appropriately sized pipette and/or the application of a burst of piezzo vibration and repeated pipetting. The size of the injection pipette is dependent upon the size of the cell. Alternatively, transfer of the nucleus may be achieved by cell fusion. As soon as possible after enucleation a single cell was placed into contact with the oocyte by using a glass pipette to transfer the cell through the hole previously made in the zona pellucida. The cytoplast/cell couplet was then transferred into fusion medium (0.3 M D-Sorbitol supplemented with 0.1mM Mg SO₄ & 0.05 mM CaCl₂ in H₂O, osmolality 280mOsM) in the fusion chamber. The couplet was manually aligned between the electrodes. Fusion was induced by application of two DC pulses of 1kV/cm for 60 µsec at intervals of 5 sec. The couplets were then washed in calcium free medium supplemented with 10% FCS at 37°C and incubated in the same medium under oil at 37°C 5% CO₂. 30 minutes prior to activation the couplets were transferred to calcium free medium 10% FCS containing 5µM Nocodazole. Activation was induced as described below, following activation the reconstructed zygotes were incubated in medium NCSU23, 10% FCS at 37°C 5% CO₂ for a further 3 hours. They were then washed 3 times for 5 minutes at 37°C in the same medium without nocodazole and cultured for a further period of time sufficient for the formation of a pronucleus prior to being used as nuclear donors for the second stage nuclear transfer embryo reconstruction. Alternatively, the donor nucleus may be transferred by either manual or piezzo aided injection or by any other chemical or physical means of producing cell fusion.

### 2. "GOAT" (G0/G1 ACTIVATION AND TRANSFER).

Enucleated oocytes were returned to the maturation medium. At 30 or 42 hours post onset of maturation a single cell was placed into contact with the enucleated oocyte. The couplet was transferred to the fusion chamber (see below) in fusion medium 0.3 M D-Sorbitol supplemented with0.1mM Mg SO₄ & 0.05 mM CaCl₂ in H₂O, osmolality 280mOsM. Fusion and activation were induced by application of two DC pulses of 1kV/cm for 60 µsec at intervals of 5 sec. Couplets were then washed in NCSU23 10% FCS and incubated at 37°C 5% CO₂ for a further period of time sufficient for the formation of a pronucleus prior to being used as nuclear donors for the second stage nuclear transfer embryo reconstruction. Alternatively, the donor nucleus may be transferred by either manual or piezzo aided injection or by any other chemical or physical means of producing cell fusion.

### 3. "UNIVERSAL RECIPIENT".

Enucleated oocytes were activated (as described below) and cultured in NCSU23, 10% FCS at 37°C 5% CO₂. After the decay of MPF activity, a single cell was then placed into contact with the oocyte and fusion induced as described below. The couplets were then cultured in NCSU23 10% FCS at 37°C 5%CO₂ for a further period of time sufficient for the formation of a pronucleus prior to being used as nuclear donors for the second stage nuclear transfer embryo reconstruction. Alternatively, the donor nucleus may be transferred by either manual or piezzo aided injection or by any other chemical or physical means of producing cell fusion.

### PREPARATION OF A CYTOPLAST FOR THE SECOND NUCLEAR TRANSFER EMBRYO RECONSTRUCTION.

A suitable cytoplast may be prepared by activation of an enucleated oocyte as described in the 'UNIVERSAL RECIPIENT'. Alternatively, as is preferred at present, a cytoplast recipient may be prepared from any *in vivo* or *in vitro* produced zygote. After pronuclear formation the zygote may be centrifuged, in a suitable buffered medium, at 13.000xg for 5 minutes, this aids visualisation of the pronuclei but is not essential. The centrifuged zygotes are placed into the manipulation chamber in a suitable medium (as above) and a portion of cytoplasm containing the two pronuclei aspirated using a micropipette.

### SECOND STAGE NUCLEAR TRANSFER EMBRYO RECONSTRUCTION.

### Synchronisation procedure for the collection of in vivo fertilised 1-cell porcine zygotes.

A suitable procedure for the collection *of in vivo* matured porcine oocytes is described below. In practice any procedure which results in the production of suitable oocytes may be used.

Feed Altrenogest (Regu-Mate, 18 mg/day, 9 AM) for 5 - 9 days, starting on d 12-16 of the cycle (day 0 is heat). If no heats are available, feed Altrenogest for 14 days. Inject gilts with Estrumate (IM, 1.0 ml, 9 AM) during the last Alternogest feeding. Inject eCG (Equine Chorionic Gonadotropin, 1500 IU IM) 14 hrs (11 PM) after the last Altrenogest feeding. Inject hCG (human Chorionic Gonadotropin, 1000 IU, IM) 79 hrs after the eCG injection (6 AM). Artificially inseminate 24 and 36 hrs after hCG injection. Collect embryos 50 hrs after the hCG injection (noon).

### Reconstruction of the second stage nuclear transfer embryo.

The karyoplast formed by aspiration of the pronucleus surrounded by a portion of cytoplasm and membrane bound from the first nuclear transfer embryo is placed below the zona pellucida of the second recipient cytoplast in contact with the cytoplast. Fusion of the karyoplast and cytoplast are carried out as below or by any other means. Alternatively the karyoplast, the pronucleus or the pronucleus surrounded by a portion of cytoplasm may be injected into the second nuclear transfer embryo.

### Culture of reconstructed embryos.

Reconstructed embryos may be cultured *in vitro* using any suitable culture medium and conditions e.g. NCSU medium supplemented with 10%FCS 37°C in a humidified atmosphere of 5.0% CO₂. Alternatively, as is presently the method of choice in porcine species the reconstructed embryos may be transferred directly to a synchronised final recipient for development to term. The transfer of embryos to a synchronised recipient has been determined to support good embryo and foetal development. In control experiments porcine zygotes were sham manipulated to mimic the zona pellucida damage occurring during the nuclear transfer procedure. These manipulated embryos were then transferred to a suitable recipient. Subsequently the recipient was sacrificed and development of the transferred embryos assessed (table 2).

**Table 2. Embryo development and pregnancy induction using in vivo derived micromanipulated porcine zygotes. Two holes were made by enucleation pipette in zona pellucida to mimic nuclear transfer micromanipulation. The embryos were then transferred to the recipients.**

| **No. of transferred "manipulated" 1cell embryos** | **Results** |
|---|---|
| 48 | 26 compacted morulae, and 15 16-cell embryos were collected on Day 6. They were cultured in NCSU-23 for 24hrs. 29 embryos developed to blastocyst stage. |
| 34 | Open - Slaughter/Pregnancy Check (SPC) on day 21 |
| 35 | Pregnant, SPC on day 21. Four fetuses and 5 reabsorbtion sites were observed. No corpus luteus (CL's), new follicles were present. Pregnancy would be lost. |

Manipulated embryos were able to develop to blastocysts and healthy foetuses.

### Fusion and Activation

For activation, oocytes were placed between two parallel electrodes in activation medium 0.3 M D-Sorbitol supplemented with0.1mM Mg SO₄ & 0.05 mM CaCl₂ in H₂O, osmolality 280mOsM. Activation was induced by application of two DC pulses of 1kV/cm for 60 µsec at intervals of 5 sec. This activation procedure promotes development of parthenogenetic embryos to the blastocyst stage (see Table 3). However, any other suitable medium may be used for example 0.3 M mannitol, 0.1mM MgSO₄, 0.001mM CaCl₂ in distilled water (Willadsen, 1986). Any suitable activation pulse may be used for instance 1 DC pulse of 1.25 kV/cm for 80µs. For fusion manipulated embryos were treated in a similar manner with the addition that the contact surface between the enucleated oocyte and the cell was arranged parallel to the electrodes. Fusion was induced by application of an AC. current of 3V for 5 seconds followed by DC pulses of 1.0 kV/cm for 60µs at 5 second intervals.

**Table 3. Development to the blastocyst stage of porcine oocytes electrically activated at different ages.**

| **Oocyte age. Hrs post hCG** | **51.5 hrs (%)** | **54 hrs (%)** | **57 hrs (%)** | **60 hrs (%)** |
|---|---|---|---|---|
| **1 experiment** | 1/13 (8) | 0/13 | 0/13 (0) | 2/14 (14) |
| **2 experiment** | 7/16 (44) | 5/16 (31) | 8/16 (50) | 8/16 (50) |
| **Average** | 8/29 (28) | 5/29 (17) | 8/29 (28) | 10/30 (33) |

### Embryo culture and assessment (all groups)

Following reconstruction embryos may be cultured by a variety of techniques known to those skilled in the art including; *in vitro* culture in a suitable medium or *in vivo* culture in the ligated oviduct of a suitable recipient host until a stage for transfer to a final surrogate recipient. Alternatively, as is presently the method of choice in porcine species although not essential, the second stage reconstructed embryos may be transferred directly to the oviduct of a suitable synchronised final recipient host for development to term (see tables 4 and 5).

**Table 4. Development of double nuclear transfer porcine embryos first stage nuclear embryos were reconstructed by pizzo injection of diploid primary fibroblasts into in vivo matured enucleated porcine oocyte, second stage nuclear transfer used in vivo produced porcine zygotes. Reconstructed embryos were transferred to the oviduct of a synchronised recipient sow and recovered on day 6 to assess development**

| **Group** | **Number attempted fusions** | **Number Lysed (%)** | **Number Fused (%)** | **Number of Blastocysts (%)** |
|---|---|---|---|---|
| 1 | 23 - fusions | 0 (0) | 13/23 (57) | 4 (31) |

**Table 5: Pregnancy outcome following transfer of double nuclear transfer embryos directly to a synchronised final recipient.**

| **Replicate** | **Number Of Embryos Transferred** | **Pregnancy And Comments** |
|---|---|---|
| 1 | 48 | Pregnant d28, open d34 |
| 2 | 4 | Open d28, Open d35 |
| 3 | 4 | Open d28, Open d35 |
| 4 | 9 | Not tested |

### Synchronisation procedure for the preparation of surrogate recipients for nuclear transfer reconstructed embryos.

Same method as used for the preparation of zygotes with the following differences, no eCG, no AI and 1.5 days behind.

### Procedure for the maintenance of pregnancy in surrogate recipients.

In order to maintain pregnancy in the final recipient *in vivo* produced embryos at the same developmental stage as the reconstructed embryos may be transferred with the nuclear transfer embryos. Alternatively, as is presently preferred in porcine species the final recipient may be treated hormonally to maintain pregnancy in the following manner, eCG (1000 IU) on day 9 or day 10 of pregnancy, hCG (500 IU) 3 to 3.5 days later.

### II) PROTOCOL FOR OVINE NUCLEAR TRANSFER:

### Materials and methods.

### Superstimulation of donor ewes and recovery of oocytes.

Scottish Blackface ewes were synchronised with progestagen sponges for 14 days (Veramix, Upjohn, UK) and induced to superovulate with single injections of 3.0 mg/day (total 6.0 mg) ovine follicle-stimulating hormone (FSH) (Ovagen, Immunochemical Products Ltd. New Zealand) on - two successive days. Ovulation was induced with an 8 mg single dose of a gonadotropin-releasing hormone analogue (GnRH Receptal, Hoechst. UK.) 24 hours after the second injection of FSH.
Unfertilised metaphase II oocytes were recovered by flushing from the oviduct at 24-29 hours after GnRH injection using Dulbecco's phosphate buffered saline containing 1.0% foetal calf serum (FCS) maintained at 37°C until use.

### Oocyte manipulation.

Recovered oocytes were maintained at 37°C, washed in PBS 1.0 % FCS and transferred to calcium free M2 medium containing 10% Foetal Calf Serum (FCS), at 37°C. To remove the chromosomes (enucleation) oocytes were placed in calcium free M2 containing 10% FCS, 7.5 µg/ml Cytochalasin B (Sigma) (Cytochalasin B is optional) and 5.0 µg/ml Hoechst 33342 (Sigma) at 37°C for 20 minutes. A small amount of cytoplasm from directly beneath the first polar body was then aspirated using a 20 µM glass pipette. Enucleation was confirmed by exposing the aspirated portion of cytoplasm to UV light and checking for the presence of a metaphase plate.

### FIRST STAGE NUCLEAR TRANSFER EMBRYO RECONSTRUCTION.

A range of protocols are available for the first stage nuclear transfer. Their use is dictated by the cell cycle stage of the donor cell nucleus at the time of transfer.

### Embryo Reconstruction.

Groups of 10-20 oocytes were enucleated and placed into 20µl drops of calcium free M2 medium at 37°C 5%CO₂ under mineral oil (SIGMA). Three protocols were used for embryo reconstruction.

### 1. "MAGIC" (METAPHASE ARRESTED G1/G0 ACCEPTING CYTOPLAST).

As soon as possible after enucleation a single cell was placed into contact with the oocyte by using a glass pipette to transfer the cell through the hole previously made in the zona pellucida. The cytoplast/cell couplet was then transferred into the fusion chamber in 200 µl of 0.3M mannitol in distilled water and manually aligned between the electrodes. An AC pulse of 5V was applied for 3 seconds followed by 3 D.C. pulses of 1.25kV/cm for 80µsecs. The couplets were then washed in calcium free M2, 10 % FCS at 37°C and incubated in the same medium under oil at 37°C 5 % CO_{2.} 30 minutes prior to activation the couplets were transferred to calcium free M2 medium 10% FCS containing 5µM Nocodazole. Activation was induced at 32-34 hours post hCG injection as described below. Following activation the reconstructed zygotes were incubated in medium TC199 10% FCS at 37°C 5% CO₂ for a further 3 hours. They were then washed 3 times for 5 minutes at 37°C in the same medium without nocodazole and cultured for a further period of time sufficient for the formation of a pronucleus prior to being used as nuclear donors for the second stage nuclear transfer embryo reconstruction. Alternatively, the donor nucleus may be transferred by either manual or piezzo aided injection or by any other chemical or physical means of producing cell fusion.

### 2. "GOAT"(G0 /G1 ACTIVATION AND TRANSFER).

At 32-34 hours post hCG injection a single cell was placed into contact with the enucleated oocyte. The couplet, was transferred to the fusion chamber (see below) in 200µl of 0.3 M mannitol, 0.1mM MgSO₄, 0.001mM CaCl₂ in distilled water. Fusion and activation were induced by application of an AC. pulse of 3V for 5 seconds followed by 3 DC. pulses of 1.25kV/Cm for 80µsecs. Couplets were then washed in TC199 10% FCS (the addition of 7.5 µg/ml Cytochalasin B to this medium is optional) and incubated in this medium for 1 hour at 37°C 5% CO₂. Couplets were then washed in TC 199 10% FCS and cultured in TC199 10% FCS at 37°C 5% CO₂. for a further period of time sufficient for the formation of a pronucleus prior to being used as nuclear donors for the second stage nuclear transfer embryo reconstruction. Alternatively, the donor nucleus may be transferred by either manual or piezzo aided injection or by any other chemical or physical means of producing cell fusion.

### 3. "UNIVERSAL RECIPIENT".

Enucleated oocytes were activated (as described below) 32-34 hours post hCG injection and then cultured in TC199 10% FCS at 37°C 5% CO₂ for 4-6 hours. A single cell was then placed into contact with the oocyte and fusion induced as described below. The couplets were then incubated in TC199 10% FCS 7.5µg cytochalasin B for 1 hour at 37°C 5%CO₂. Couplets were then washed and cultured in TC199 10% FCS at 37°C 5% CO₂ for a further period of time sufficient for the formation of a pronucleus prior to being used as nuclear donors for the second stage nuclear transfer embryo reconstruction. Alternatively, the donor nucleus may be transferred by either manual or piezzo aided injection or by any other chemical or physical means of producing cell fusion.

### SECOND STAGE NUCLEAR TRANSFER EMBRYO RECONSTRUCTION.

### Collection of zygotes.

### Superstimulation of donor ewes and recovery of oocytes.

Scottish Blackface ewes were synchronised with progestagen sponges for 14 days (Veramix, Upjohn, UK) and induced to superovulate with single injections of 3.0 mg/day (total 6.0 mg) ovine follicle-stimulating hormone (FSH) (Ovagen, Immunochemical Products Ltd. New Zealand) on two successive days. Ovulation was induced with an 8 mg single dose of a gonadotropin-releasing hormone analogue (GnRH Receptal, Hoechst. UK.) 24 hours after the second injection of FSH.
Unfertilised metaphase II oocytes were recovered by flushing from the oviduct at 24-29 hours after GnRH injection using Dulbecco's phosphate buffered saline containing 1.0% foetal calf serum (FCS) maintained at 37°C until use. Recovered oocytes were fertilised *in vitro* using techniques available to those skilled in the art. Alternatively superstimulated ewes were mated or fertilised by artificial insemination using fresh or frozen serum. Fertilised zygotes were recovered surgically by flushing from the oviduct in any suitable medium.

### Preparation of a cytoplast for the second nuclear transfer embryo reconstruction.

A suitable cytoplast may be prepared by activation of an enucleated oocyte as described in the 'UNIVERSAL RECIPIENT'. Alternatively, as is preferred at present, a cytoplast recipient may be prepared from any *in vivo* or *in vitro* produced zygote. After pronuclear formation the zygote may be centrifuged, in a suitable buffered medium, at 13.000xg for 5 minutes, this aids visualisation of the pronuclei but is not essential. The centrifuged zygotes are placed into the manipulation chamber in a suitable medium (as above) and a portion of cytoplasm containing the two pronuclei aspirated using a micropipette.

### RECONSTRUCTION OF THE SECOND STAGE NUCLEAR TRANSFER EMBRYO.

The karyoplast formed by aspiration of the pronucleus surrounded by a portion of cytoplasm and membrane bound from the first nuclear transfer embryo is placed below the zona pellucida of the second recipient cytoplast in contact with the cytoplast. Fusion of the karyoplast and cytoplast are carried out as below or by any other means. Alternatively the karyoplast, the pronucleus or the pronucleus surrounded by a portion of cytoplasm may be injected into the second nuclear transfer embryo.

### Fusion and Activation

For activation, oocytes were placed between two parallel electrodes in 200µl of 0.3 M mannitol, 0.1mM MgSO₄, 0.001mM CaCl₂ in distilled water (Willadsen, 1986). Activation was induced by application of 1 DC pulse of 1.25 kV/cm for 80µs. For fusion manipulated embryos were treated in a similar manner with the addition that the contact surface between the enucleated oocyte and the cell was arranged parallel to the electrodes. Fusion was induced by application of an AC. current of 3V for 5 seconds followed by 3 DC pulses of 1.25 kV/cm for 80 µs

### Embryo culture and assessment (all groups)

After the culture period fused couplets were double embedded in 1% and 1.2% agar (DIFCO) in PBS (or any other suitable protective covering material) and transferred to the ligated oviduct of unsynchronised ewes. After 6 days recipient ewes were sacrificed and the embryos retrieved by flushing from the oviduct using PBS 10% FCS. Embryos were dissected from the agar chips using 2 needles and development assessed by microscopy. All embryos which had developed to the morula/blastocyst stage were transferred as soon as possible to the uterine horn of synchronised final recipient ewes. Alternatively the second stage reconstructed embryos may be transferred directly to the oviduct of a suitable synchronised final recipient host for development to term.

### III) PROTOCOL FOR BOVINE NUCLEAR TRANSFER:

### MATERIALS AND METHODS.

Bovine oocytes and zygotes for use as recipients for nuclear transfer embryo reconstruction may be obtained from any source or by any of the methods available to anyone skilled in the art. The following examples are non-limiting and are merely representative of various aspects of the present invention.

### In vitro oocyte maturation

Ovaries were obtained from a local abattoir and maintained at 28 - 32°C during transport to the laboratory. Cumulus oocyte complexes (COC's) were aspirated from follicles 3 - 10mm in diameter using a hypodermic needle (1.2mm internal diameter) and placed into sterile plastic universal containers. The universal containers were placed into a warmed chamber (35°C) and the follicular material allowed to settle for 10 - 15 minutes before pouring off three quarters of the supernatant. The remaining follicular material was diluted with an equal volume of dissection medium (TCM 199 with Earles salts( Gibco), 75.0 mg/l kanamycin, 30.0mM Hepes, pH7.4, osmolarity 280 mOsmols/Kg H₂O) supplemented with 10% bovine serum , transferred into an 85mm petri dish and searched for COC's under a dissecting microscope. Complexes with at least 2-3 compact layers of cumulus cells were selected washed three times in dissection medium and transferred into maturation medium (TC medium 199 with Earles salts (Gibco), 75mg/l kanamycin, 30.0mM Hepes, 7.69mM NaHCO₃, pH7.8, osmolarity 280mOsmols/Kg H₂O) supplemented with 10% bovine serum and 1x10⁶ granulosa cells /ml and cultured until required on a rocking table at 39°C in an atmosphere of 5% CO₂ in air.

### Oocyte manipulation.

Matured oocytes were stripped of cumulus cells 18 hours after the onset of maturation. Denuded oocytes were then washed in calcium free M2 medium containing 10% Foetal Calf Serum (FCS) and maintained in this medium at 37°C. To remove the chromosomes (enucleation) oocytes were placed in calcium free M2 containing 10% FCS, 7.5 ug/ml Cytochalasin B (Sigma) and 5.0 ug/ml Hoechst 33342 (Sigma) at 37°C for 20 minutes. A small amount of cytoplasm from directly beneath the first polar body was then aspirated using a 20 µM glass pipette. Enucleation was confirmed by exposing the aspirated portion of cytoplasm to UV light and checking for the presence of a metaphase plate. Enucleated oocytes were then used for each of the three methods of reconstruction as detailed below.

### FIRST STAGE NUCLEAR TRANSFER EMBRYO RECONSTRUCTION.

A range of protocols are available for the first stage nuclear transfer. Their use is dictated by the cell cycle stage of the donor cell nucleus at the time of transfer.

### 1. "MAGIC" (METAPHASE ARRESTED G1/G0 ACCEPTING CYTOPLAST).

Enucleated oocytes were maintained in calcium free M2 10% FCS at 39°C as soon as possible after enucleation a single cell was placed into contact with the oocyte by using a glass pipette to transfer the cell through the hole previously made in the zona pellucida. The cytoplast/cell couplet was then transferred into the fusion chamber in 200 µl of 0.3M mannitol in distilled water. The couplet was manually aligned between the electrodes. An AC pulse of 3V was applied for 5 seconds followed by 3 D.C. pulses of 1.25kV/cm for 80µsecs. The couplets were then washed in calcium free M2, 10% FCS at 37°C and incubated in the same medium under oil at 37°C 5% CO₂ 30 minutes prior to activation the couplets were transferred to calcium free M2 medium 10% FCS containing 5µM Nocodazole. Activation was induced as described below, following activation the reconstructed zygotes were incubated in medium TC199 10% FCS at 37°C 5% CO₂ for a further 3 hours. They were then washed 3 times for 5 minutes at 37°C in the same medium without nocodazole and cultured for a further period of time sufficient for the formation of a pronucleus prior to being used as nuclear donors for the second stage nuclear transfer embryo reconstruction. Alternatively, the donor nucleus may be transferred by either manual or piezzo aided injection or by any other chemical or physical means of producing cell fusion.

### 2. "GOAT" (G0/G1 ACTIVATION AND TRANSFER).

Enucleated oocytes were returned to the maturation medium. At 30 or 42 hours post onset of maturation a single cell was placed into contact with the enucleated oocyte. The couplet was transferred to the fusion chamber (see below) in 200µl of 0.3 M mannitol, 0.1mM MgSO₄, 0.001mM CaCl₂ in distilled water. Fusion and activation were induced by application of an AC. pulse of 3V for 5 seconds followed by 3 DC. pulses of 1.25kV/Cm for 80µsecs. Couplets were then washed in TC199 10% FCS and incubated at 37°C 5% CO₂ for a further period of time sufficient for the formation of a pronucleus prior to being used as nuclear donors for the second stage nuclear transfer embryo reconstruction. Alternatively, the donor nucleus may be transferred by either manual or piezzo aided injection or by any other chemical or physical means of producing cell fusion.

### 3. "UNIVERSAL RECIPIENT".

Enucleated oocytes were activated (as described below) 30 or 42 hours post onset of maturation and then cultured in TC199 10% FCS at 37°C 5% CO₂ for 8-10 hours (30 hpm group) or 4-6 hours (42 hpm group). A single cell was then placed into contact with the oocyte and fusion induced as described below. The couplets were then cultured in TC199 10% FCS at 37°C 5%CO₂ for a further period of time sufficient for the formation of a pronucleus prior to being used as nuclear donors for the second stage nuclear transfer embryo reconstruction. Alternatively, the donor nucleus may be transferred by either manual or piezzo aided injection or by any other chemical or physical means of producing cell fusion.

### SECOND STAGE NUCLEAR TRANSFER EMBRYO RECONSTRUCTION. Preparation of a cytoplast for the second nuclear transfer embryo reconstruction.

A suitable cytoplast may be prepared by activation of an enucleated oocyte as described in the 'UNIVERSAL RECIPIENT'. Alternatively, as is preferred at present, a cytoplast recipient may be prepared from any *in vivo or in vitro* produced zygote. After pronuclear formation the zygote may be centrifuged, in a suitable buffered medium, at 13.000xg for 5 minutes, this aids visualisation of the pronuclei but is not essential. The centrifuged zygotes are placed into the manipulation chamber in a suitable medium (as above) and a portion of cytoplasm containing the two pronuclei aspirated using a micropipette.

### Reconstruction of the second stage nuclear transfer embryo.

The karyoplast formed by aspiration of the pronucleus surrounded by a portion of cytoplasm and membrane bound from the first nuclear transfer embryo is placed below the zona pellucida of the second recipient cytoplast in contact with the cytoplast. Fusion of the karyoplast and cytoplast are carried out as below or by any other means. Alternatively the karyoplast, the pronucleus or the pronucleus surrounded by a portion of cytoplasm may be injected into the second nuclear transfer embryo.

### Fusion and Activation

For activation, oocytes were placed between two parallel electrodes in 200µl of 0.3 M mannitol, 0.1mM MgSO₄, 0.001mM CaCl₂ in distilled water (Willadsen, 1986). Activation was induced by application of 1 DC pulse of 1.25 kV/cm for 80µs. For fusion manipulated embryos were treated in a similar manner with the addition that the contact surface between the enucleated oocyte and the cell was arranged parallel to the electrodes. Fusion was induced by application of an AC. current of 3V for 5 seconds followed by 3 DC pulses of 1.25 kV/cm for 80 µs.

### Embryo culture and assessment (all groups)

After the second stage nuclear transfer fused couplets were double embedded in 1 % and 1.2% agar (DIFCO) in PBS and transferred to the ligated oviduct of synchronised or unsynchronised ewes. After 6 days recipient ewes were sacrificed and the embryos retrieved by flushing from the oviduct using PBS 10% FCS. Embryos were dissected from the agar chips using 2 needles and development assessed by microscopy. Alternatively the second stage reconstructed embryos may be transferred directly to the oviduct of a suitable synchronised final recipient host for development to term.

**Table 1. Possible cell cycle combinations of donor and recipient cells for first NT in order to maintain ploidy of the nucleus/i of the reconstructed zygotes.**

| **DONOR CELL CYCLE STAGE** | **RECIPIENT CYTOPLAST** | **OTHER TREATMENT** |
|---|---|---|
| G0 | MII CONCOMITANT with ACTIVATION | |
| G0 | MII POST-ACTIVATION | CHEMICAL MAINTENANCE OF PLOIDY (i.e. Nocodazole, Colcemid, DMAP or other serine threonine kinase inhibitors). |
| G0 | MII PRE-ACTIVATION | |
| G1 | MII CONCOMITANT with ACTIVATION | |
| G1 | MII POST-ACTIVATION | CHEMICAL MAINTENANCE OF PLOIDY (i.e. Nocodazole, Colcemid, DMAP, or other serine threonine kinase inhibitors). |
| G1 | MII PRE-ACTIVATION | |
| S | PRE-ACTIVATION | |
| G2 | MII enucleated POST-ACTIVATION | Tetraploid PN Transfer to late G2/ mitotic 1 cell zygote |
| G2 | MII unenucleated POST-ACTIVATION | Subsequent enucleation *Suppression of PB formation if required to form 2 diploid pronuclei |
| G2 | MII PRE-ACTIVATION | |
| S | MII PRE-ACTIVATION | |
| M | MII enucleated POST-ACTIVATION | Depolymerisation of spindle if required i.e nocodazole to form a single tetraploid pronucleus. Transfer to late G2/mitotic I cell zygote |
| M | MII enucleated PRE-ACTIVATION LATE G2 (MPF rising or high) | |
| M | MII enucleated POST-ACTIVATION | Tetraploid PN Transfer to late G2/mitotic 1 cell zygote |
| M | MII unenucleated POST-ACTIVATION | Subsequent enucleation *Suppression of PB formation if required to form 2 diploid pronuclei |

| | | |
|---|---|---|
| *Dependent upon species. | | |

### IV. GENERATION OF CLONED PIGS USING SOMATIC CELLS AS NUCLEAR DONORS.

### Isolation of porcine granulosa cells:

Granulosa cells were harvested from ovarian follicles by needle aspiration of follicular fluid. Animals from which ovaries were collected were prepared identically to those used for oocyte collection (see Oocyte recovery below). Follicles were aspirated by penetrating through the cortex of the ovary using an 18 gauge hypodermic needle attached to a 10 ml syringe. Follicular fluid was collected from follicles between 2 and 5 mm in diameter, was placed into a 15ml conical centrifuge tube, and centrifuged at 1000 rpm for 10 min. The cell pellet was resuspended into DMEM (Gibco), containing 10% FCS (Summit Biotech), NEAA, 2 ng/ml bFGF and 6 µl/ml gentamycin. Cells were expanded for several days and then cryopreserved.

### Preparation of cells for nuclear transfer.

Donor cells were plated at 1-5 X 10⁴ cells per 35 mm dish and were allowed to reach 100% confluency in DMEM medium supplemented with NEAA (0.1 mM), bFGF (2ng/ml), and 10% fetal calf serum. Contact inhibition was chosen for cell synchronization in order to maximize the number of healthy cells with a diploid DNA content. See cell cycle data for details.

Cells were harvested by trypsinisation and stored in suspension in a calcium-free phosphate buffered version of NCSU-23 medium at 38.5 °C for 20-120 minutes, prior to use as nuclear donors.

### Cell cycle analysis (granulosa cells)

Since MII oocytes were used as recipient cytoplasts for the first step of nuclear transfer (NT), donor cells having a diploid DNA content were chosen to maintain the appropriate coordination of ploidy of the resultant reconstructed embryo. The position within the cell cycle of various populations of cells after 8 days of culture, was determined by measuring the DNA content using flow cytometry. Three populations were analyzed: sub-confluent actively growing granulosa cells, contact inhibited granulosa cells, and granulosa cells serum starved for 48 hours.
The results of this experiment are summarized in Table 6. It was observed that following serum starvation, the granulosa cell population contained a large proportion (7.2%) of structures having a DNA content lower than that consistent with a cell in G1 (they were sub-G1). These structures are characteristic of cells undergoing apoptosis and senescence, and thus indicated that the granulosa cells did not tolerate serum starvation very well. Synchronization of the cells by contact inhibition (100% confluent) was more effective, given that a higher proportion of cells were in G1/G0 (90.3%), and there were fewer apoptotic sub-G1 cells in the population (only 1.6%).

**Table 6. Cell cycle analysis of granulosa cells. Values are expressed as the percentage of the total number of cells in the population.**

| Stage Of Cell Cycle | Non-Starved | Serum Starved | 100% Confluent |
|---|---|---|---|
| G1/G0 | 78.8 | 82.3 | 90.3 |
| S | 3.7 | 1.9 | 2.2 |
| G2/M | 13.9 | 6.7 | 4.5 |
| Sub G1 | 0.7 | 7.2 | 1.6 |

### Synchronization and superovulation of gilts for oocyte and zygote recovery

### Oocyte recoveries.

Crossbred gilts (280-320 lbs.) were synchronized, superovulated and oocytes were collected. Gilts were synchronized by oral administration of 18-20 mg Regu-Mate (Altrenogest, Hoechst) mixed into the feed. Regu-Mate was fed for 5-14 days using a scheme that is dependent on the stage of the estrous cycle. Estrumate (250 µg, Bayer) was administered IM the last day of the Regu-Mate treatment. Superovulation was induced with a 1600 IU IM injection of Pregnant Mare Serum Gonadotropin (PMSG, Diosynth) 15-17 hours after the last Regu-Mate feeding. 1500 units of human Chorionic Gonadotropin (hCG, Intervet America) were administered IM 82 hours after the PMSG injection. Oocytes were collected 50-52 hours after the hCG injection using Dulbecco's phosphate buffered saline containing BSA (4 g/l).

### Pregnancy maintenance

Pregnancy was maintained by using a combination of Pregnant Mare Serum Gonadotropin (PMSG) and Human Chorionic Gonadotropin (hCG). PMSG (1000 IU) was injected IM on d10 of the estrous cycle, d1 being the day of estrus. Human Chorionic Gonadotropin was injected IM 3 to 3.5 days later, on d13 of the cycle.

### Zygote recoveries.

Crossbred gilts (280-320 lbs.) were synchronized, superovulated and zygotes were collected. Gilts were synchronized by oral administration of 18-20 mg Regu-Mate (Altrenogest, Hoechst) mixed into the feed. Regu-Mate was fed for 5-14 days; dependent on the stage of the estrous cycle the gilts were in. Estrumate (250 g, Bayer) was administered IM the last day of the Regu-Mate treatment. Superovulation was induced by a 1600 IU IM injection of Pregnant Mare Serum Gonadotropin (PMSG, Diosynth) 15-17 hours after the last Regu-Mate feeding. 1500 units of Human Chorionic Gonadotropin (hCG, Intervet America) were administered 78 hours after the PMSG injection. The gilts were then either artificially inseminated or bred naturally 24-36 hours after the hCG injection. Zygotes were collected 52-54 hours after the hCG injection using Dulbecco's phosphate buffered saline containing BSA (4 g/l).

### Oocyte enucleation.

Recovered oocytes were maintained at 38°C, washed in phosphate buffered saline (PBS) with 4 g/l BSA and transferred to calcium free phosphate buffered NCSU-23 medium, at 38°C. To remove the chromosomes (enucleation) oocytes were first placed in calcium free phosphate buffered NCSU-23 medium containing 5 µg/ml Cytochalasin B (Sigma) (Cytochalasin B is optional) and 7.5 µg/ml Hoechst 33342 (Sigma) at 38°C for 20 minutes. A small amount of cytoplasm from directly beneath the 1st polar body was then aspirated using a 18 µM glass pipette. Enucleation was confirmed by exposing the aspirated portion of cytoplasm in the pipette to UV light, and checking for the presence of a metaphase plate.

### Embryo reconstruction (Day 1).

Groups of 10-20 oocytes were enucleated and placed into 20µl drops of calcium free phosphate buffered version of the NCSU-23 medium at 38°C under mineral oil (SIGMA). At 53-56 hours post hCG injection, a single cell was placed under the zona pellucida in contact with the enucleated cytoplasm. The couplet was transferred to a fusion chamber (model # BT-453, BTX Inc. San Diego, CA) containing 700µl of 0.3 M mannitol, 0.1mM MgS0₄, 0.1mM CaCl₂ in deionized water. Fusion and activation were induced by application of an AC pulse of 5V for 5 seconds followed by 2 DC pulses of 1.5kV/Cm for 60µsec using an ECM2001 Electrocell Manipulator (BTX Inc., San Diego, CA). Couplets were then washed in bicarbonate buffered NCSU-23 medium, and incubated in this medium for 0.5-1 hour at 38.6°C in a humidified atmosphere consisting of 5% CO₂ in air. Couplets were then checked for fusion at 300X magnification using an inverted microscope. Fused reconstructed embryos were artificially activated using 2 successive DC pulses of 1.2kV/cm for 60µsec each, and cultured overnight.

### Double Nuclear Transfer (Second day)

The double nuclear transfer procedure refers to the reconstruction of enucleated zygotes on day 2, using nuclei produced by day 1 nuclear transfer.

### Assessment of nuclear formation from previous day (day 1) of nuclear transfer :

Reconstructed embryos from previous day of nuclear transfer were removed from culture and centrifuged in a Biofuge at 13,000 rpm for 15 min to observe nucleus formation. Embryos were evaluated for the presence of a pseudo-pronucleus by observation at 300X using an inverted microscope. Embryos with a pseudo-pronucleus were sorted, counted, and placed back in an NCSU-23-containing culture plate in an incubator at 38.6°C, with 5% CO² in air.

### Zygote Enucleation:

Zygotes were centrifuged at 13,000 rpm for 15 min in Biofuge 13 centrifuge. The zygotes were then placed in phosphate buffered NCSU-23 medium containing 5.0µg/ml Cytochalasin B (Sigma) at 38°C, and incubated for 20 minutes.incubated for 20 minutes. Fertilized zygotes with 2 pronuclei were used for enucleation.

To remove the nuclei, an enucleation pipette (25-35 µm) was inserted through the zona pelucida and into the cytoplasm, so that the opening of the enucleation pipette was close to the pronuclei. Both pronuclei and the polar bodies were aspirated by slowly applying negative pressure on the enucleation line.

### Reconstruction of Enucleated Zygotes:

Day 1 reconstructed embryos were placed into phosphate buffered NCSU-23 medium containing Cytochalasin B and Nocodazole. A 30-45 µm enucleation pipette was used for manipulation. Karyoplasts were prepared by aspirating the psuedo-pronucleus with minimal surrounding cytoplasm. These karyoplast were slowly expeled into the perivitelline space of enucleated zygotes. The couplets were transferred to the fusion chamber containing 700µl of SOR2 medium. Fusion was induced by application of an AC pulse of 5V for 5 seconds followed by 2 DC pulses of 1.2kV/cm for 60µsec. Couplets, were then washed with NCSU-23 medium, incubated in this medium for 0.5-1 hour at 38.6°C, 5% CO₂, and then checked for fusion. Fused couplets were transferred as soon as possible to the oviduct of an estrus-synchronized recipient gilt. Results of nuclear transfer using primary granulosa cells are summarized in Table 7.

**Table 7. Nuclear transfer using porcine granulosa cells**

| | |
|---|---|
| No. reconstructed embryos - Day 1 | 196 |
| No. fused embryos - Day 1 (%) | 124/183 (68) |
| No. reconstructed embryos - Day 2 | 74 |
| No. fused embryos - Day 2 (%) | 72/74 (97) |
| No. embryos transferred to recipient | 72 |
| No. fetuses detected at day 29 by ultrasound scan | 3¹ |
| No. fetuses detected at day 42 by ultrasound scan | 2² |

| | |
|---|---|
| ¹ at least 3 fetuses; ² at least 2 fetuses. It was difficult to determine the exact number of the fetuses by ultrasound in pigs due to the anatomical position of the pig uterus. | |

Ultrasound images confirmed the pregnancies at day 29 and day 42 of gestation.

### PROTOCOL FOR BOVINE NUCLEAR TRANSFER:

### MATERIALS AND METHODS.

Bovine oocytes and zygotes for use as recipients for nuclear transfer embryo reconstruction may be obtained from any source or by any of the methods available to anyone skilled in the art. The following examples are non-limiting and are merely representative of various aspects of the present invention.

### In vitro oocyte maturation

Ovaries were obtained from a local abattoir and maintained at 28 - 32°C during transport to the laboratory. Cumulus oocyte complexes (COC's) were aspirated from follicles 3 - 10mm in diameter using a hypodermic needle (1.2mm internal diameter) and placed into sterile plastic universal containers. The universal containers were placed into a warmed chamber (35°C) and the follicular material allowed to settle for 10 - 15 minutes before pouring off three quarters of the supernatant. The remaining follicular material was diluted with an equal volume of dissection medium ( TCM 199 with Earles salts( Gibco), 75.0 mg/l kanamycin, 30.0mM Hepes, pH7.4, osmolarity 280 mOsmols/Kg H₂O) supplemented with 10% bovine serum , transferred into an 85mm petri dish and searched for COC's under a dissecting microscope. Complexes with at least 2-3 compact layers of cumulus cells were selected washed three times in dissection medium and transferred into maturation medium (TC medium 199 with Earles salts (Gibco), 75mg/l kanamycin, 30.0mM Hepes, 7.69mM NaHCO₃, pH7.8, osmolarity 280mOsmols/Kg H₂O) supplemented with 10% bovine serum and 1x10⁶ granulosa cells /ml and cultured until required on a rocking table at 39°C in an atmosphere of 5% CO₂ in air. Early during maturation 13 hours post onset the oocytes wer placed into medoium containing Hoescht 3332 (Sigma) 1µg /ml for 10 minutes, washed 3 times in maturation medium without Hoescht and returned to maturation. Alternatively the donor cells were exposed to growth medium containing Hoescht 3332) 1µg /ml for 10 minutes then washed 3 times in growth medium and stored in culture medium until use.

### Oocyte manipulation.

Matured oocytes were stripped of cumulus cells 18 hours after the onset of maturation. Denuded oocytes were then washed in calcium free M2 medium containing 10% Foetal Calf Serum (FCS) and maintained in this medium at 37°C.

### UNENUCLEATED OOCYTE RECONSTRUCTION.

oocytes were maintained in calcium free M2 10% FCS at 39°C a single cell was placed into contact with the oocyte by using a glass pipette to transfer the cell through the hole previously made in the zona pellucida. The cytoplast/cell couplet was then transferred into the fusion chamber in 200 µl of 0.3M mannitol in distilled water. The couplet was.manually aligned between the electrodes. An AC pulse of 3V was applied for 5 seconds followed by 3 D.C. pulses of 1.25kV/cm for 80µsecs. The couplets were then washed in calcium free M2, 10% FCS at 37°C and incubated in the same medium under oil at 37°C 5% CO₂ for 60 minutes. Fusion was assessed microscopically. Fused couplets were placed into maturation medium for 2 -20 hours. Alternatively, the donor nucleus may be transferred by either manual or piezzo aided injection or by any other chemical or physical means of producing cell fusion.

### ENUCLEATION:

1. Fused oocytes were incubated as described above. Following incubation the maternal chromatin was removed. To remove the chromosomes (enucleation) oocytes were placed in calcium free M2 containing 10% FCSat 37°C. If the 1^{st} polar body was visible a small amount of cytoplasm from directly beneath the 1st polar body was then aspirated using a 20 µM glass pipette. If the polar body was not visible the oocyte was exposed briefly 1-2 seconds to UV light to identify the stained donor (or oocyte) chromatin.
2. At 24-45 hours post onset of maturation oocytes were activated as described below. 30-60 minutes following activation ( or sufficient time for telophase to occur) the relevant chromatin was identified by brief exposure to UV light and removed by aspiration
3. At 24 -45 hours following the onset of maturation, activation was induced as described below, following activation the reconstructed zygotes were incubated in medium TC199 10% FCS at 37°C 5% CO₂ for a further period of time sufficient for the formation of a pronucleus. The relevant pronucleus was identified by brief exposure to UV light and aspirated

Enucleated oocytes were returned to the maturation medium. At 30 or 42 hours post onset of maturation a single cell was placed into contact with the enucleated oocyte. The couplet was transferred to the fusion chamber (see below) in 200µl of 0.3 M mannitol, 0.1mM MgSO₄, 0.001mM CaCl₂ in distilled water. Fusion and activation were induced by application of an AC. pulse of 3V for 5 seconds followed by 3 DC. pulses of 1.25kV/Cm for 80µsecs. Couplets were then washed in TC199 10% FCS and incubated at 37°C 5% CO₂ for a further period of time sufficient for the formation of a pronucleus prior to being used as nuclear donors for the second stage nuclear transfer embryo reconstruction. Alternatively, the donor nucleus may be transferred by either manual or piezzo aided injection or by any other chemical or physical means of producing cell fusion.

### Fusion and Activation

For activation, oocytes were placed between two parallel electrodes in 200µl of 0.3 M mannitol, 0.1mM MgSO₄, 0.001mM CaCl₂ in distilled water (Willadsen, 1986). Activation was induced by application of 1 DC pulse of 1.25 kV/cm for 80µs. For fusion manipulated embryos were treated in a similar manner with the addition that the contact surface between the enucleated oocyte and the cell was arranged parallel to the electrodes. Fusion was induced by application of an AC. current of 3V for 5 seconds followed by 3 DC pulses of 1.25 kV/cm for 80 µs

### Embryo culture and assessment (all groups)

After nuclear transfer reconstructed embryos may be cultured by any method known to the art these include but are not limited to ;
1. Embryos were double embedded in 1% and 1.2 %agar(DIFCO) in PBS and transferred to the ligated oviduct of synchronised or unsynchronised ewes. After 6 days recipient ewes were sacrificed and the embryos retrieved by flushing from the oviduct using PBS 10% FCS. Embryos were dissected from the agar chips using 2 needles and development assessed by microscopy.
2. Alternatively the reconstructed embryos may be transferred directly to the oviduct of a suitable synchronised final recipient host for development to term.
3. Alternatively embryos may be cultured *in vitro* by any suitable means to a stage suitable for transfer to a synchronized recipient.

## Claims

1. A method of reconstituting a non-human animal embryo, the process comprising transferring a non-human nucleus into a non-human first oocyte followed by removing and transferring said nucleus from said oocyte to a further non-human oocyte or to an enucleated fertilised non-human zygote.

2. A method as claimed in claim 1, wherein the first oocyte is a mature metaphase II oocyte (unfertilised egg) or an activated MII oocyte.

3. A method as claimed in claim 1 or claim 2, wherein the further oocyte is an enucleated MII oocyte.

4. A method as claimed in any one of claim 1 to 3, in which the reconstructed embryo is cultured *in vitro* or in vivo to a stage suitable for transfer to a final surrogate recipient for development to term.

5. A method as claimed in any one of claims 1 to 3, in which the reconstructed embryo is transferred to a final surrogate recipient to support embryo development and development to term.

6. A method as claimed in any one of claims 1 to 5, in which the donor nucleus is genetically modified.

7. A method as claimed in any of claims 1 to 6, wherein a diploid cell donates the nucleus.

8. A method as claimed in any one claims 1-7, wherein the cell is a somatic cell.

9. A method as claimed in any one of claims 1 to 8, wherein the diploid nucleus is donated by a G1 cell.

10. A method as claimed in any one of claims 1 to 8, wherein the diploid nucleus is donated by a cell arrested at the G1/S-phase border.

11. A method as claimed in any one of claims 1 to 6, wherein the nucleus is donated by a tetraploid cell.

12. A method as claimed in claim 11, wherein the tetraploid nucleus is donated by a G2 cell.

13. A method was claimed in claim 11 or claim 12, wherein the tetraploid nucleus is donated by a mitotic cell.

14. A method as claimed in any one of claims 1 to 6, wherein the nucleus is donated by a cell of unknown ploidy.

15. A method as claimed in claim 14, wherein the nucleus of unknown ploidy is donated by a growing cell at any point in the cell cycle i. e. G1. S, G2 or M.

16. A method as claimed in any one of claims 1 to 6, wherein the nucleus is donated by a cell arrested at any point in the cell cycle i. c. GO, G1, G1/S, S, G2 or M by any means

17. A method as claimed in any one of claimed to 16. wherein the recipient oocyte for the first nuclear transfer is enucleate.

18. A method as claimed in any one of claims 1 to 17, wherein the first nuclear transfer is achieved by cell fusion, or by cell or nuclear injection.

19. A. method as claimed in any one of claims 1 to 18. in which the animal is an ungulate species.

20. A method as claimed in any one or claims 1 to 19, wherein the animal is a cow or bull, pig, sheep, goat, camel or water buffalo.

21. A method as claimed is any one of claims 1 to 18, wherein the animal is a mouse, rat or other rodent.

22. A method as claimed in any one of claims 1 to 18, wherein the animal is a lagomorph.

23. A method as claimed in claim 22, wherein the animal is a rabbit.

24. A method as claimed in any one of claims 1 to 23, wherein correct ploidy is maintained by combination of donor and recipient cell cycle stage.

25. A method as claimed in any one of claims 1 to 24, wherein correct ploidy is maintained by treatment of the reconstructed embryo with any compound/s that maintain correct ploidy.

26. The method as claimed in claim 25, wherein the compound is Nocodazole.

27. The method as claimed in claim 25, wherein the compound is Colcemid.

28. The method as claimed in claim 25, wherein the compound is DMAP.

29. The method as claimed in claim 25, wherein the compound is a serine threonine kinase inhibitor.

30. A method as claimed in any of the claims 1 to 29, wherein the donor nucleus is then transferred to a non-human second recipient cell.

31. A method as claimed in any claim 1 to 30 wherein the second recipient cell is a fertilised zygote.

32. A method as claimed in any claim 1 to 30 wherein the second recipient cell is an activated oocyte.

33. A method as claimed in claim 32, wherein the activated oocyte is activated by chemical or physical means.

34. A method as claimed in claim 33, wherein the chemical or physical activation is by a treatment that induces calcium entry into the oocyte or release of internal calcium stores.

35. A method as claimed in claim 33 or claim 34, wherein the chemical activation is hy treatment with ethanol, ionomycin, inositol tris-phosphate calcium ionophore A23187.

36. A method as claimed in claim 33 or claim 34, wherein the chemical activation is by treatment with extracts of sperm.

37. A method as claimed in claim 33 or claim 34, wherein the physical activation is by application of a DC electrical stimulus.

38. A method as claimed in any one of claims 33 to 37, wherein the chemical or physical activation further comprises treatment with inhibitors of protein synthesis or inhibitor of serine threonine protein kinases.

39. A method as claimed in any of the claims 1 to 38 wherein the second recipient cell is enucleated.

40. A method of preparing a non-human animal the method comprising :
(a) reconstituting a non-human animal embryo as claimed in any one of the preceding claims;
(b) causing a foetus to develop from the embryo; and
(c) causing a non-human animal to develop to term from the foetus; and
(d) optionally, breeding from the non-human animal so formed.

41. A method as claimed in claim 40, wherein the non-human animal embryo is further manipulated prior to full development of the embryo.

42. A method as claimed in claim 40, wherein the non-human animal foetus is further manipulated prior to full development of the foetus.

43. A method as claimed in any one of claims 40 to 42, wherein a new cell line is derived from the reconstructed embryo.

44. A method as claimed in any one of claims 40 to 42, wherein a new cell line is derived from the non-human animal foetus prior to full development of the embryo.

45. A method as claimed in any one of claims 40 to 42, wherein a new cell line is derived from the resultant non-human animal.

46. A method claimed in any one of claims 40 to 45, wherein more than one non-human animal is derived from the embryo.

47. A method as claimed in any one of claims 40 to 46, wherein the non-human animal is a pig.

## Patentansprüche

1. Verfahren zum Wiederherstellen eines nicht-menschlichen Tierembryos, wobei das Verfahren das Übertragen eines nicht-menschlichen Zellkerns in eine nicht-menschliche erste Oozyte umfaßt, gefolgt von einem Entfernen und Übertragen des Zellkerns von dieser Oozyte auf eine weitere nicht-menschliche Oozyte oder einer entkernten befruchteten nicht-menschlichen Zygote.

2. Verfahren nach Anspruch 1, wobei die erste Oozyte eine reife Metaphase II Oozyte (unbefruchtetes Ei) oder eine aktivierte MII Oozyte ist.

3. Verfahren nach Anspruch 1 oder Anspruch 2, wobei die weitere Oozyte eine entkernte MII Oozyte ist.

4. Verfahren nach einem der Ansprüche 1 bis 3, in welchem der wiederhergestellte Embryo *in vitro* oder *in vivo* kultiviert wird bis zu einem Stadium, welches für den Transfer auf einen endgültigen Ersatzempfänger für eine Entwicklung bis zu Niederkunft geeignet ist.

5. Verfahren nach einem der Ansprüche 1 bis 3, in dem der wiederhergestellte Embryo auf einen endgültigen Ersatzempfänger übertragen wird, um die Entwicklung des Embryos und die Entwicklung bis zur Niederkunft zu unterstützen.

6. Verfahren nach einem der Ansprüche 1 bis 5, in dem der Spenderzellkern genetisch modifiziert wird.

7. Verfahren nach einem der Ansprüche 1 bis 6, wobei eine diploide Zelle den Zellkern spendet.

8. Verfahren nach einem der Ansprüche 1 bis 7, wobei die Zelle eine somatische Zelle ist.

9. Verfahren nach einem der Ansprüche 1 bis 8, wobei der diploide Zellkern von einer G1-Zelle gespendet wird.

10. Verfahren nach einem der Ansprüche 1 bis 8, wobei der diploide Zellkern von einer Zelle gespendet wird, die an der G1/S-Phasengrenze arretiert ist.

11. Verfahren nach einem der Ansprüche 1 bis 6, wobei der Zellkern von einer tetraploiden Zelle gespendet wird.

12. Verfahren nach Anspruch 11, wobei der tetraploide Zellkern von einer G2-Zelle gespendet wird.

13. Verfahren nach Anspruch 11 oder Anspruch 12, wobei der tetraploide Zellkern von einer mitotischen Zelle gespendet wird.

14. Verfahren nach einem der Ansprüche 1 bis 6, wobei der Zellkern von einer Zelle mit einer unbekannten Ploidie gespendet wird.

15. Verfahren nach Anspruch 14, wobei der Zellkern mit unbekannter Ploidie von einer wachsenden Zelle an jedem Punkt des Zellzyklusses, d.h. G1, S, G2 oder M, gespendet wird.

16. Verfahren nach einem der Ansprüche 1 bis 6, wobei der Zellkern von einer Zelle, die an jedem Punkt des Zellzyklusses, d.h. G0, G1, G1/S, S, G2 oder M, durch jedwede Mittel arretiert wird, gespendet wird.

17. Verfahren nach einem der Ansprüche 1 bis 16, wobei die Empfänger-Oozyte für den ersten Zellkerntransfer entkernt wird.

18. Verfahren nach einem der Ansprüche 1 bis 17, wobei der erste Zellkerntransfer durch Zellfusion oder durch Zell- oder Zellkern-Injektion erreicht wird.

19. Verfahren nach einem der Ansprüche 1 bis 18, wobei das Tier eine Huftierart ist.

20. Verfahren nach einem der Ansprüche 1 bis 19, wobei das Tier eine Kuh oder ein Bulle, ein Schwein, ein Schaf, eine Ziege, ein Kamel oder ein Wasserbüffel ist.

21. Verfahren nach einem der Ansprüche 1 bis 18, wobei das Tier eine Maus, eine Ratte oder ein weiteres Nagetier ist.

22. Verfahren nach einem der Ansprüche 1 bis 18, wobei das Tier ein Hasenartiges ist.

23. Verfahren nach Anspruch 22, wobei das Tier ein Kaninchen ist.

24. Verfahren nach einem der Ansprüche 1 bis 23, wobei die korrekte Ploidie durch eine Kombination der Zellzyklusphasen von Spender und Empfänger beibehalten wird.

25. Verfahren nach einem der Ansprüche 1 bis 24, wobei die korrekte Ploidie durch das Behandeln des wiederhergestellten Embryos mit jeglichen Verbindung(en), welche die korrekte Ploidie beibehalten, beibehalten wird.

26. Verfahren nach Anspruch 25, wobei die Verbindung Nocodazol ist.

27. Verfahren nach Anspruch 25, wobei die Verbindung Colcemid ist.

28. Verfahren nach Anspruch 25, wobei die Verbindung DMAP ist.

29. Verfahren nach Anspruch 25, wobei die Verbindung ein Serinthreoninkinase - Inhibitor ist.

30. Verfahren nach einem der Ansprüche 1 bis 29, wobei der Spenderzellkern anschließend auf eine nicht-menschliche zweite Empfängerzelle übertragen wird.

31. Verfahren nach einem der Ansprüche 1 bis 30, wobei die zweite EmpfängerZelle eine befruchtete Zygote ist.

32. Verfahren nach einem der Ansprüche 1 bis 30, wobei die zweite Empfängerzelle eine aktivierte Oozyte ist.

33. Verfahren nach Anspruch 32, wobei die aktivierte Oozyte durch chemische oder physikalische Mittel aktiviert wird.

34. Verfahren nach Anspruch 33, wobei die chemische oder physikalische Aktivierung durch eine Behandlung, welche einen Kalziumeintritt in die Oozyte oder eine Freisetzung von inneren Kalziumvorräten induziert, geschieht.

35. Verfahren nach Anspruch 33 oder Anspruch 34, wobei die chemische Aktivierung durch die Behandlung mit Ethanol, lonomycin, Inositol-tris-phosphat, Kalziumionophore A23187 geschieht.

36. Verfahren nach Anspruch 33 oder Anspruch 34, wobei die chemische Aktivierung durch eine Behandlung mit Spermaextrakten geschieht.

37. Verfahren nach Anspruch 33 oder Anspruch 34, wobei die physikalische Aktivierung durch die Anwendung eines elektrischen Gleichstrom-Stimulus geschieht.

38. Verfahren nach einem der Ansprüche 33 bis 37, wobei die chemische oder physikalische Aktivierung weiter die Behandlung mit Inhibitoren der Proteinsynthese oder Inhibitoren von Serinthreonin-Proteinkinasen umfaßt.

39. Verfahren nach einem der Ansprüche 1 bis 38, wobei die zweite Empfängerzelle entkernt ist.

40. Verfahren zur Herstellung eines nicht-menschlichen Tieres, wobei das Verfahren
(a) das Wiederherstellen eines nicht-menschlichen Tierembryos, wie in einem der vorhergehenden Ansprüche definiert;
(b) das Verursachen der Entwicklung eines Fötus aus dem Embryo; und
(c) das Verursachen der Entwicklung eines nicht-menschlichen Tieres aus dem Fötus bis zur Niederkunft; und
(d) gegebenenfalls, das Züchten des so gebildeten nicht-menschlichen Tieres
umfaßt.

41. Verfahren nach Anspruch 40, wobei der nicht-menschliche Tierembryo vor der vollständigen Entwicklung des Embryos weiter manipuliert wird.

42. Verfahren nach Anspruch 40, wobei der nicht-menschliche Tierfötus vor der vollständigen Entwicklung des Fötus weiter manipuliert wird.

43. Verfahren nach einem der Ansprüche 40 bis 42, wobei eine neue Zelllinie von dem wiederhergestellten Embryo abgeleitet wird.

44. Verfahren nach einem der Ansprüche 40 bis 42, wobei die neue Zelllinie von dem nicht-menschlichen Fötus vor der vollständigen Entwicklung des Embryos abgeleitet wird.

45. Verfahren nach einem der Ansprüche 40 bis 42, wobei die neue Zelllinie von dem resultierenden nicht-menschlichen Tier abgeleitet wird.

46. Verfahren nach einem der Ansprüche 40 bis 45, wobei mehr als ein nichtmenschliches Tier aus dem Embryo abgeleitet wird.

47. Verfahren nach einem der Ansprüche 40 bis 46, wobei das nicht-menschliche Tier ein Schwein ist.

## Revendications

1. Procédé de reconstitution d'un embryon animal non humain, le procédé comprenant le transfert d'un noyau non humain dans un premier ovocyte non humain suivi par le retrait et le transfert dudit noyau depuis le dit ovocyte vers un autre ovocyte non humain ou vers un zygote non humain fertilisé énucléé.

2. Procédé selon la revendication 1, dans lequel le premier ovocyte est un ovocyte en métaphase II mature (oeuf non fertilisé) ou un ovocyte en MII activé.

3. Procédé selon la revendication 1 ou la revendication 2, dans lequel l'autre ovocyte est un ovocyte en MII énucléé.

4. Procédé selon l'une quelconque des revendications 1 à 3, dans lequel l'embryon reconstruit est mis en culture *in vitro* ou *in vivo* jusqu'à un stade approprié pour un transfert vers un receveur porteur final pour un développement à terme.

5. Procédé selon l'une quelconque des revendications 1 à 3, dans lequel l'embryon reconstruit est transféré vers un receveur porteur final pour permettre le développement de l'embryon et le développement à terme.

6. Procédé selon l'une quelconque des revendications 1 à 5, dans lequel le noyau donneur est génétiquement modifié.

7. Procédé selon l'une quelconque des revendications 1 à 6, dans lequel une cellule diploïde donne le noyau.

8. Procédé selon l'une quelconque des revendications 1 à 7, dans lequel la cellule est une cellule somatique.

9. Procédé selon l'une quelconque des revendications 1 à 8, dans lequel le noyau diploïde est donné par une cellule en phase G1.

10. Procédé selon l'une quelconque des revendications 1 à 8, dans lequel le noyau diploïde est donné par une cellule arrêtée à la transition G1/S.

11. Procédé selon l'une quelconque des revendications 1 à 6, dans lequel le noyau est donné par une cellule tétraploïde.

12. Procédé selon la revendication 11, dans lequel le noyau tétraploïde est donné par une cellule en phase G2.

13. Procédé selon la revendication 11 ou la revendication 12, dans lequel le noyau tétraploïde est donné par une cellule mitotique.

14. Procédé selon l'une quelconque des revendications 1 à 6, dans lequel le noyau est donné par une cellule de ploïdie inconnue.

15. Procédé selon la revendication 14, dans lequel le noyau de ploïdie inconnue est donné par une cellule en croissance à un stade quelconque du cycle cellulaire, à savoir G1, S, G2 ou M.

16. Procédé selon l'une quelconque des revendications 1 à 6, dans lequel le noyau est donné par une cellule arrêtée à un stade quelconque du cycle cellulaire, à savoir G0, G1, G1/S, S, G2 ou M par un moyen quelconque.

17. Procédé selon l'une quelconque des revendications 1 à 16, dans lequel l'ovocyte receveur pour le premier transfert nucléaire est énucléé.

18. Procédé selon l'une quelconque des revendications 1 à 17, dans lequel le premier transfert nucléaire est effectué par fusion cellulaire, ou par injection cellulaire ou nucléaire.

19. Procédé selon l'une quelconque des revendications 1 à 18, dans lequel l'animal est une espèce ongulée.

20. Procédé selon l'une quelconque des revendications 1 à 19, dans lequel l'animal est une vache ou un taureau, un porc, un mouton, une chèvre, un chameau ou un buffle des Indes.

21. Procédé selon l'une quelconque des revendications 1 à 18, dans lequel l'animal est une souris, un rat ou autre rongeur.

22. Procédé selon l'une quelconque des revendications 1 à 18, dans lequel l'animal est un lagomorphe.

23. Procédé selon la revendication 22, dans lequel l'animal est un lapin.

24. Procédé selon l'une quelconque des revendications 1 à 23, dans lequel une ploïdie correcte est maintenue par combinaison de la phase de cycle cellulaire du donneur et du receveur.

25. Procédé selon l'une quelconque des revendications 1 à 24, dans lequel une ploïdie correcte est maintenue par traitement de l'embryon reconstruit avec un ou plusieurs composés quelconques qui maintiennent une ploïdie correcte.

26. Procédé selon la revendication 25, dans lequel le composé est le Nocodazole.

27. Procédé selon la revendication 25, dans lequel le composé est la Colcémide.

28. Procédé selon la revendication 25, dans lequel le composé est la DMAP.

29. Procédé selon la revendication 25, dans lequel le composé est un inhibiteur de la sérine thréonine kinase.

30. Procédé selon l'une quelconque des revendications 1 à 29, dans lequel le noyau donneur est ensuite transféré vers une seconde cellule receveuse non humaine.

31. Procédé selon l'une quelconque des revendications 1 à 30, dans lequel la seconde cellule receveuse est un zygote fertilisé.

32. Procédé selon l'une quelconque des revendications 1 à 30, dans lequel la seconde cellule receveuse est un ovocyte activé.

33. Procédé selon la revendication 32, dans lequel l'ovocyte activé est activé par des moyens chimiques ou physiques.

34. Procédé selon la revendication 33, dans lequel l'activation chimique ou physique se fait par un traitement qui déclenche l'entrée de calcium dans l'ovocyte ou la libération de réserves internes de calcium.

35. Procédé selon la revendication 33 ou la revendication 34, dans lequel l'activation chimique se fait par traitement avec de l'éthanol, de l'ionomycine, de l'inositol tris-phosphate, de l'ionophore de calcium A23187.

36. Procédé selon la revendication 33 ou la revendication 34, dans lequel l'activation chimique se fait par traitement avec des extraits de sperme.

37. Procédé selon la revendication 33 ou la revendication 34, dans lequel l'activation physique se fait par application d'un stimulus électrique CC.

38. Procédé selon l'une quelconque des revendications 33 à 37, dans lequel l'activation chimique ou physique comprend en outre un traitement avec des inhibiteurs de la synthèse des protéines ou des inhibiteurs des sérine thréonine protéine, kinases.

39. Procédé selon l'une quelconque des revendications 1 à 38, dans lequel la seconde cellule receveuse est énucléée.

40. Procédé de préparation d'un animal non humain, le procédé comprenant de :
(a) reconstituer un embryon animal non humain selon l'une quelconque des revendications précédentes ;
(b) provoquer le développement d'un foetus à partir de embryon ; et
(c) provoquer le développement à terme d'un animal non humain à partir du foetus ; et
(d) facultativement, effectuer des croisements à partir de l'animal non humain ainsi formé.

41. Procédé selon la revendication 40, dans lequel l'embryon animal non humain est en outre manipulé avant le développement complet de l'embryon.

42. Procédé selon la revendication 40, dans lequel le foetus d'animal non humain est en outre manipulé avant le développement complet du foetus.

43. Procédé selon l'une quelconque des revendications 40 à 42, dans lequel une nouvelle lignée cellulaire est dérivée de l'embryon reconstruit.

44. Procédé selon l'une quelconque des revendications 40 à 42, dans lequel une nouvelle lignée cellulaire est dérivée du foetus d'animal non humain avant le développement complet de l'embryon.

45. Procédé selon l'une quelconque des revendications 40 à 42, dans lequel une nouvelle lignée cellulaire est dérivée de l'animal non humain résultant.

46. Procédé selon l'une quelconque des revendications 40 à 45, dans lequel plus d'un animal non humain est dérivé de l'embryon.

47. Procédé selon l'une quelconque des revendications 40 à 46, dans lequel l'animal non humain est un porc.
